# EUROPEAN PATENT APPLICATION

(11) **EP 4 745 985 A1**
(43) Date of publication of application: **20.05.2026**
(21) Application number: 25215402.6
(22) Date of filing: 12.11.2025
(51) Int. Cl.: G16H 40/20, G16H 50/70, G16H 30/40

(54) **DEVICES, SYSTEMS, AND METHODS FOR OPTIMIZING SCHEDULING AND INVENTORY ASSOCIATED WITH MEDICAL PROCEDURES**

(30) Priority: 14.11.2024 US 202463720546 P
(71) Applicant: Mako Surgical Corporation, Weston, Florida 33331 (US)
(72) Inventor: Williamson, Tom, Fort Lauderdale, 33331 (US); Fox, Amber, Fort Lauderdale, 33331 (US)
(74) Representative: Purdylucey Intellectual Property

(57) **Abstract**

Systems and methods are disclosed for receiving a surgical schedule and data associated with one or more surgical robots; receiving one or more input parameters; executing a predictive model, stored in the computer-readable storage medium, to optimize the surgical schedule based on the data associated with the one or more surgical robots and the one or more input parameters; generating an updated surgical schedule based on the predictive model; and generating and presenting an electronic display of the updated surgical schedule.

## Description

### FIELD OF THE DISCLOSURE

This disclosure relates to systems and methods for optimizing scheduling and inventory associated with medical procedures, and in particular to a system and a method for determining real-time and predicted scheduling and inventory efficiencies associated with surgical robots, among other aspects.

### BACKGROUND OF THE DISCLOSURE

Planning surgical procedures logistically typically involves coordination of numerous hospital resources. A traditional model for supporting and managing surgeries that involve medical devices includes may include surgical planning, inventory planning, intra-operative support, and post-surgery support. During the surgical planning phase, a surgeon may contact the hospital where the surgery is to be performed to schedule resources such as operating rooms, inventory, and support staff. Hospitals typically maintain a surgery schedule using an internal enterprise resource planning system that includes information such as the type of surgery, the name of the surgeon, the date the surgery is to be performed, and the supply and equipment needs of the surgery. Furthermore, hospitals may be limited in the number of surgeries they can provide based on the available inventory at present and in the future, particularly where the inventory includes surgical robots and guidance carts.

As such, there remains a need for improved real-time and predictive surgery planning and inventory control.

### BRIEF SUMMARY OF THE DISCLOSURE

In an aspect of the present disclosure, the techniques described herein relate to a computer-implemented method comprising: receiving, by one or more processors, a surgical schedule and optionally data associated with one or more surgical robots; receiving, by one or more processors, one or more input parameters; executing a predictive model, stored in a non-transitory computer-readable storage medium, to optimize a surgical schedule based on the data associated with the one or more surgical robots and the one or more input parameters; generating, by the one or more processors, an updated surgical schedule based on the predictive model; and generating and presenting, by the one or more processors, an electronic display of the updated surgical schedule.

In any embodiment, the predictive model is configured to maximize daily operating time per surgical robot.

In any embodiment, the input parameters include one or more difficulty of procedure parameters for one or more procedures, the one or more difficulty of procedure parameters being determined by CT imaging data.

In any embodiment, the input parameters include one or more parameters indicating a stage of a surgical procedure.

In any embodiment, the method further comprises:
receiving prior procedure data, the prior procedure data including data from a plurality of prior patients sharing at least one characteristic with a current patient;
and determining a predicted duration for a medical procedure based on the received prior procedure data.

In any embodiment, the method further comprises:
receiving at least one of (i) patient specific data regarding the current patient, (ii) clinical data relating to the current patient, and (iii) surgeon specific data relating to one or more surgeons, wherein determining the predicted duration for the medical procedure is based on the received patient specific data, clinical data, and/or surgeon specific data.

In any embodiment, the method further comprises:
determining a predicted duration for a medical procedure of a current patient; and
determining, based on the predicted duration for the medical procedure and/or at least one parameter of the current patient's anatomy, an output, the output including at least one of:
   an operating room layout,
   an operating room schedule, or
   at least one staff member to assist in performance of the medical procedure.

In any embodiment, the method further comprises:
determining, based on the predicted duration for the medical procedure and/or the at least one parameter of the current patient's anatomy, an output, the output including at least one of:
   a risk of infection,
   a loss of cartilage,
   a predicted pain perceived by the patient after the procedure,
   a predicted stress level perceived by the patient after the procedure,
a predicted anxiety level perceived by the patient after the procedure, or
a predicted mental health status of the patient after the procedure.

In any embodiment, the method further comprises:
receiving, by the one or more processors, the surgical schedule including one or more medical procedures at one or more facilities;
receiving, by the one or more processors, data associated with a surgical tool;
executing the predictive model, stored in the non-transitory computer-readable storage medium, to predict scheduling and inventory requirements based on the surgical schedule and the data associated with the surgical tool;
generating, by the one or more processors, an updated surgical schedule and updated inventory requirements based on the predictive model; and
generating and presenting an electronic display of the updated surgical schedule and the updated inventory requirements.

In some aspects, the techniques described herein relate to a system for optimizing scheduling and inventory associated with medical procedures, comprising: a computer-readable storage medium storing instructions for generating and presenting an electronic display of a surgical schedule; and one or more processors configured to execute the instructions to perform a method including: receiving a surgical schedule and data associated with one or more surgical robots; receiving one or more input parameters; executing a predictive model, stored in the computer-readable storage medium, to optimize the surgical schedule based on the data associated with the one or more surgical robots and the one or more input parameters; generating an updated surgical schedule based on the predictive model; and generating and presenting an electronic display of the updated surgical schedule.

In some aspects, the techniques described herein relate to a computer-implemented method for generating scheduling and inventory predictions, the method comprising: receiving, by one or more processors, a surgical schedule including one or more medical procedures; receiving, by one or more processors, trend data associated with one or more medical procedures associated with the surgical schedule, wherein the trend data includes one or more of patient growth rate, medical procedure growth rate, and surgical robot use growth rate; executing a predictive model, stored in a non-transitory computer-readable storage medium, to predict scheduling and inventory requirements based on the surgical schedule and the trend data; generating, by the one or more processors, an updated surgical schedule and updated inventory requirements based on the predictive model; and generating and presenting an electronic display of the updated surgical schedule and optionally updated inventory requirements.

In any embodiment, the predictive model is configured to maximize daily operating time per surgical robot.

In any embodiment, the input parameters include one or more difficulty of procedure parameters for one or more procedures, the one or more difficulty of procedure parameters being determined by CT imaging data.

In any embodiment, the input parameters include one or more parameters indicating a stage of a surgical procedure.

In any embodiment, the instructions further comprise:
receiving prior procedure data, the prior procedure data including data from a plurality of prior patients sharing at least one characteristic with a current patient; and
determining a predicted duration for a medical procedure based on the received prior procedure data.

In any embodiment, the instructions further comprise:
receiving at least one of (i) patient specific data regarding the current patient, (ii) clinical data relating to the current patient, and (iii) surgeon specific data relating to one or more surgeons, wherein determining the predicted duration for the medical procedure is based on the received patient specific data, clinical data, and/or surgeon specific data.

In any embodiment, the instructions further comprise:
determining, based on the determined predicted duration for the procedure and/or at least one parameter of the patient's anatomy, an output, the output including at least one of:
an operating room layout,
an operating room schedule, or
at least one staff member to assist in performance of the medical procedure.

In any embodiment, the instructions further comprise:
determining, based on a determined predicted duration for the procedure and/or at least one parameter of the patient's anatomy, an output, the output including at least one of:
a risk of infection,
a loss of cartilage,
a predicted pain perceived by the current patient after the medical procedure,
a predicted stress level perceived by the current patient after the medical procedure,
a predicted anxiety level perceived by the current patient after the medical procedure, or
a predicted mental health status of the current patient after the medical procedure.

In any embodiment, the instructions further comprise:
receiving, by the one or more processors, a surgical schedule including one or more medical procedures at one or more facilities;
receiving, by the one or more processors, data associated with a surgical tool;
executing the predictive model, stored in the non-transitory computer-readable storage medium, to predict scheduling and inventory requirements based on the surgical schedule and the data associated with the surgical tool;
generating, by the one or more processors, an updated surgical schedule and inventory requirements based on the predictive model; and
generating and presenting an electronic display of the updated surgical schedule and inventory requirements.

In any embodiment, the one or more medical procedures include procedures using surgical robots, and the predictive model is configured to maximize daily operating time per surgical robot.

### BRIEF DESCRIPTION OF THE DRAWINGS

A more complete appreciation of the subject matter of this disclosure and the various advantages thereof may be understood by reference to the following detailed description, in which reference is made to the following accompanying drawings:
FIG. 1 is a schematic diagram depicting an electronic data processing system having a predictive scheduling model, according to aspects of this disclosure.
FIG. 2 is a schematic diagram of the electronic data processing system of FIG. 1 depicting interactions among preoperative measurement systems, preoperative data, the predicted surgical case time model, outputs, and output systems, according to aspects of this disclosure.
FIG. 3 is an exemplary flowchart of a computer-implemented or computer-based process for optimizing scheduling and inventory associated with medical procedures, according to aspects of this disclosure.
FIG. 4 shows an exemplary machine learning training flow chart, according to aspects of this disclosure.
FIG. 5 illustrates an implementation of an exemplary computer system that executes techniques presented herein, according to aspects of this disclosure.

### DETAILED DESCRIPTION

Reference will now be made in detail to the various embodiments of the present disclosure, some of which are illustrated in the accompanying drawings. Wherever possible, the same or like reference numbers will be used throughout the drawings to refer to the same or like features. It should be noted that the drawings are in simplified form and are not drawn to precise scale.

Hospitals and other medical facilities have an interest in efficiency and economy in optimizing the use of their surgical facilities and inventory, such as surgical tools, surgical robots, and guidance carts. The availability of operating rooms, staff, and tools and other equipment, dictate the ability of a facility to optimize its surgical schedules. Furthermore, surgical case types, surgeon equipment preferences, patient volume, inventory availability, staff availability, and costs per procedure are important considerations when generating a surgical schedule. A traditional model for supporting and managing surgeries that involve medical devices may include surgical planning, inventory planning, intra-operative support, and post-surgery support.

Systems, methods, and devices described herein may include an automated system for a surgical equipment use schedule and an operating schedule based on input parameters. Surgical equipment may include surgical robots and guidance carts. The system may take input parameters including, e.g., predictive surgical case types, surgeon equipment preferences, patient volume, inventory availability, staff availability, and costs per procedure, and output parameters including a surgical schedule, inventory schedule, estimated cost savings, and recommended cost saving actions, etc.

Surgical robots represent a major capital investment to medical facilities, such that optimizing use of surgical robots and/or guidance carts may lead to advantageous benefits, such as cost savings, performance of additional cases, an improvement in robot and/or system utilization, and an expansion of the benefits of robotic surgery to a wider patient cohort, among other benefits. Furthermore, optimizing schedule and inventory with real-time stage of procedure and/or difficulty of procedure included as an input parameter may allow for increased optimization of real-time surgical planning, while long-term planning tools that take into account patient and surgery growth rates to predict future inventory requirements may also be incorporated. Additionally, new products may be evaluated for incorporation into inventory based on scheduling needs and return on investment for purchasing and incorporating the new products. This disclosure is directed to systems and methods for addressing the aforementioned challenges, among other aspects.

In describing preferred embodiments of the disclosure, reference will be made to directional nomenclature used in describing the human body. It is noted that this nomenclature is used only for convenience and that it is not intended to be limiting with respect to the scope of the invention. For example, as used herein, the term "distal" means toward the human body and/or away from the operator, and the term "proximal" means away from the human body and/or towards the operator. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such system, process, method, article, or apparatus. The term "exemplary" is used in the sense of "example," rather than "ideal." Further, relative terms such as, for example, "about," "substantially," "approximately," etc., are used to indicate a possible variation of ±10% in a stated numeric value or range.

Additionally, the term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described.

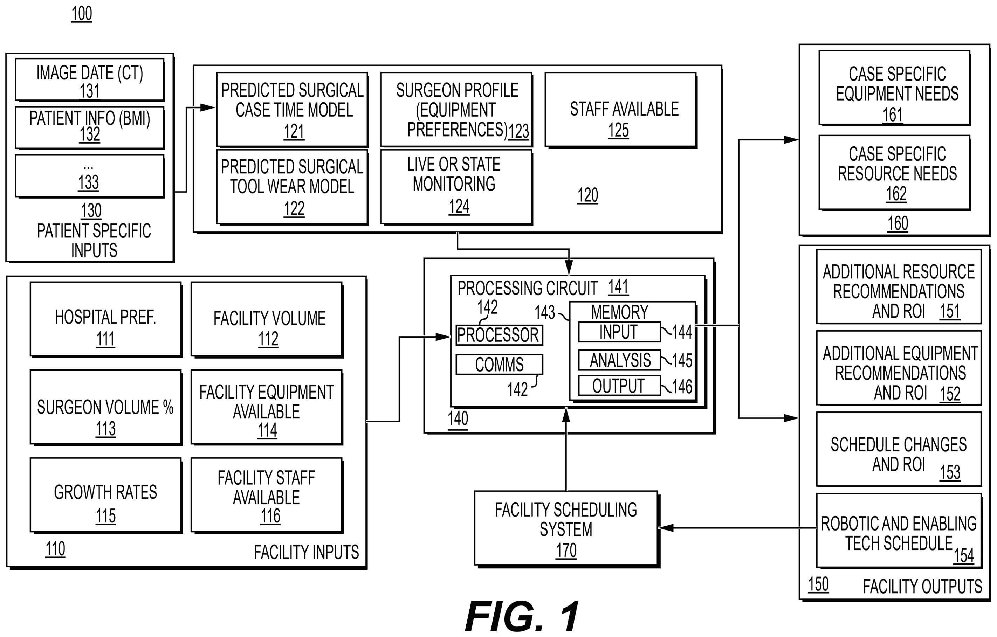

FIG. 1 illustrates a schematic electronic data processing system 100 for collecting, storing, processing, and outputting data associated with surgical planning and inventory control.

The schematic electronic data processing system 100 includes a plurality of input sources, including facility inputs 110, for example, from a hospital or other medical facility, medical device provider inputs 120, for example, from a provider of medical equipment and tools, such as surgical robots and guidance carts, and patient specific inputs 130 from a given patient that is, will be undergoing, and/or has had a surgical procedure.

The facility inputs 110 may include, for example, hospital preferences 111, which may include types of surgeries, types of equipment, etc., and further including preference of hospital in terms of parameter to be optimized for (e.g., maximize reimbursement vs minimize time vs procedure split over surgeons, etc.).

The facility inputs may also include facility volume 112, which may include a total number of operating rooms, beds, etc.. Facility volume 112 may also include a number of cases per time period performed at the facility, and the types of procedures. This may be a current volume, a current case list to be optimized, or a historical and/or future view across a larger time period, e.g., a week, month, or year, etc. In some examples, a future view of predicted volume may be determined based on historical and/or real time data.

A further facility input may include a surgeon volume percentage 113, which accounts for total amounts of surgeons that implement a given tool, e.g., a surgical robot, and may account for changing patterns of surgeon volume percentage. The surgeon volume percentage 113 may be a percentage determined by the number of surgeries the surgeon has scheduled compared to the number of surgeries the surgeon is capable of performing over a particular time period. The number of surgeries the surgeon is capable of performing may be determined by historical data, by data received from the particular surgeon and/or hospital, etc. For example, the system may identify trend data in the increasing use of surgical robots among surgeons. The surgeon volume percentage may include a variety of surgeon volume percentages for specific types of surgeries, such as a split of total procedures done per surgeon and the specific types of surgery performed. This may include surgeon preferences regarding procedure types, operating days and times, preferred staff pairings, experience level, etc.

Other facility inputs 110 may include facility equipment availability 114, which may include an inventory of all tools and equipment used in surgeries, such as surgical robots, guidance carts, surgical scalpels, saws, and other tools, and the availability of the tools and equipment at any given time, accounting for scheduled surgeries, phases of surgeries, sterilization status of tools, etc. Overall resources and equipment available including operating rooms, other adjunct technology such as mobile imaging systems (c-arms, CT scanners), endoscopy stacks, instrument trays, etc., that may be required to perform a procedure.

The facility inputs 110 may also include a value related to growth rates 115, which may be based on a predicted model and/or based on past growth rates, for example past groth rates projected into the future. The growth rates 115 may be measures of the growth of facility volume, facility equipment, number of surgeries to be demanded, number of surgeries available, or a combination of any or all of the above. Facility volume may include a total number of operating rooms, beds, etc. at a given facility, e.g., a hospital or clinic, and may also include a number of cases per time period performed at the facility. Facility equipment may include surgical robots, guidance carts, tools and their trays, and other equipment available at a facility to aid in the performance of surgical procedures. The number of surgeries demanded may be a value dependent on a variety of external factors and may be predicted into the future based on, for example, the demographics of a surrounding area of a facility, past trends extrapolated into the future, and predictions of changes based on other external factories. The number of surgeries available may be a value reflecting trends in growth in facility volume, number of surgeons available, and improvements in time per procedure based on extrapolated data or predictions based on changes in technology, etc.

A change in procedure volume over a given time period may include an increase or decrease overall (e.g. for all procedures done at the facility) and of particular procedure types, and may include demographic changes in catchment region. The growth rates 115 may include future plans such as hiring of additional surgeons or staff, facility expansion and growth, competitor status and growth rates, population increase predicted in the catchment region, etc.

The facility staff availability 116 may also be input from the facility, and may include the availability of administrative staff, support staff, etc., and may represent a combination of total number of staff available, or a number of man-hours of staff available for a given time period, e.g., a day, a week, a month, etc. This may include a list of available staff, or staff qualified to complete a specific task, to perform surgery, and may include working times and preferences, co-worker preferences, vacation and rostering information, etc.

The medical device provider inputs 120 may be inputs from a provider maintaining and offering, for example, surgical robots and guidance carts, handheld surgical devices such as drills, endoscopes, or other devices, and/or other specific tools or equipment.

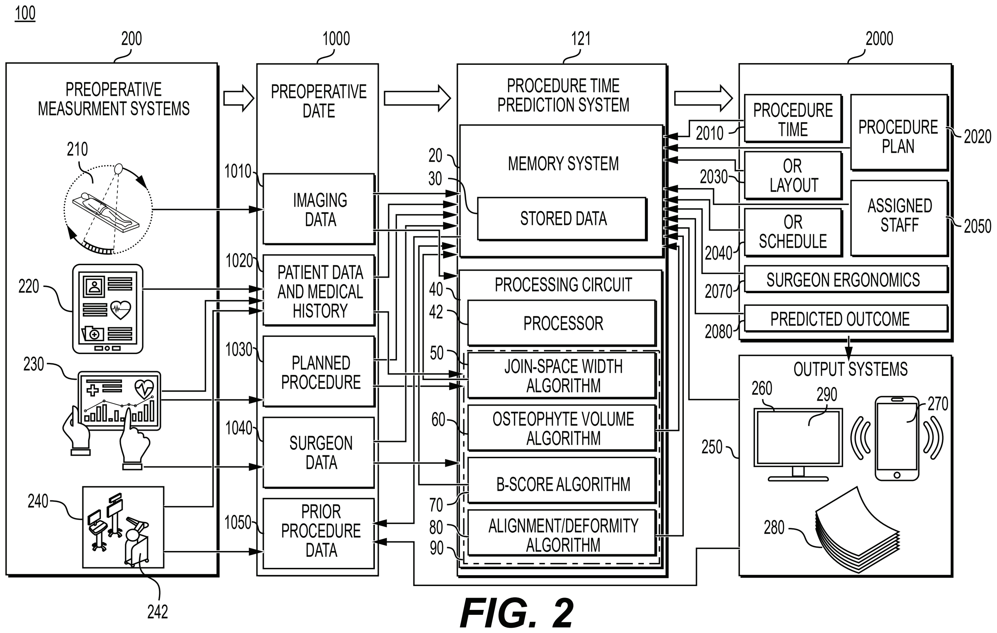

The medical device provider inputs 120 include predicted surgical case times provided by a predicted surgical case time model 121, to be described in more detail with respect to fig. 2. The predicted surgical case times may be based on a predicted difficulty of a specific procedure based on patient-specific inputs. The medical device provider inputs 120 include predicted surgical tool wear determined by a predetermined surgical tool wear model 122 that includes prediction of tool wear and case difficulty for e.g. preventative maintenance, equipment replacement, additional equipment ordering, etc. Tool wear may include a measure of device life, or device wear. For example, the tool wear includes both predictions about the lifetime of the device before it can no longer be used, and an indication of when maintenance is required for a tool, and the type of maintenance required. The medical device provider inputs 120 include a surgeon profile 123, including equipment preferences. This may include preferences of surgeons in terms of equipment and technology. For example, a surgeon may prefer a Mako robot, or a handheld device, a navigated device, and/or a traditional total knee arthroplasty. Workflow preferences of the surgeon, e.g., staff and their tasks, positioning of tools and equipment within the OR, etc., implant preferences and a surgeon's level of experience with technology may also be input. The medical device provider inputs 120 also include live operating room (OR) state monitoring inputs 124 via ambient intelligence or software state tracking. This may help determine stage or phase of an operation, e.g., a registration page or bone prep page, or a measure of percentage bone preparation complete. In some examples, the determined stage or phase of an operation may include a pre-resection phase and a post-resection phase. The medical device provider inputs 120 further include staff availability 125, including working preferences and rostering information of staff associated with the medical device provider.

The patient specific inputs may include, for example, image data 131 from a computed tomography (CT) scan or magnetic resonance imaging (MRI), to be described in more detail below, patient information 132 such as body mass index (BMI), and other patient information 133, for example, patient demographics, comorbidities, age, medical records, injury history, etc. In some examples, the patient specific inputs 130 may be input directly into the predicted surgical case time model 121 to help determine a predicted surgical case time for a given patient.

The schematic electronic data processing system 100 includes a predictive scheduling model 140. The input parameters received from the input sources, including facility inputs 110, medical device provider inputs 120, and patient specific inputs 130 may be provided to the predictive scheduling model 140. The predictive scheduling model 140 may be utilized to implement the various functions (e.g., calculations, processes, analyses) described herein. Predictive scheduling model 140 may include a processing circuit 141 having a processor 142 and memory 143. Processor 142 can be implemented as a general purpose processor, an application specific integrated circuit (ASIC), one or more field programmable gate arrays (FPGAs), a group of processing components, or other suitable electronic processing components. Memory 143 (e.g., memory, memory unit, storage device, etc.) may be one or more devices (e.g., RAM, ROM, Flash-memory, hard disk storage, etc.) for storing data and/or computer code for completing or facilitating the various processes described in the present application. Memory 143 may be or include volatile memory or non-volatile memory. Memory 143 may include database components, object code components, script components, or any other type of information structure for supporting the various activities described in the present application. According to an exemplary embodiment, memory 143 may be communicably connected to processor 142 and may include computer code for executing one or more processes described herein. The memory 143 may contain a variety of modules, each capable of storing data and/or computer code related to specific types of functions. In one embodiment, memory 143 contains several modules related to medical procedures, such as an input module 144, an analysis module 145, and an output module 146.

It should be understood that the predictive scheduling model 140 need not be contained in a single housing. Rather, components of predictive scheduling model 140 may be located in various locations of the system 100 depicted in FIG. 1, or even in a remote location. Components of predictive scheduling model 140, including components of the processor 142 and memory 143, may be located, for example, in a cloud server system or iun components of different robotic systems or in the robotic system components (e.g., in the guidance components) of an assisted procedure.

This disclosure contemplates methods, systems, and program products on any machine-readable media for accomplishing various operations. The machine-readable media may be part of or may interface with the predictive scheduling model 140. The embodiments of this disclosure may be implemented using existing computer processors, or by a special purpose computer processor for an appropriate system, incorporated for this or another purpose, or by a hardwired system. Embodiments within the scope of this disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage, other magnetic storage devices, solid state storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general purpose computer, special purpose computer, or special purpose processing machines to perform a certain function or group of functions.

Referring again to FIG. 1, the predictive scheduling model 140 further includes one or more communication interfaces 147. The communication interfaces 147 can be or include wired or wireless interfaces (e.g., jacks, antennas, transmitters, receivers, transceivers, wire terminals, etc.) for conducting data communications with external sources via a direct connection or a network connection (e.g., an Internet connection, a LAN, WAN, or WLAN connection, etc.). For example, communication interfaces 28 can include an Ethernet card and port for sending and receiving data via an Ethernet-based communications link or network. In another example, communication interfaces 147 can include a Wi-Fi transceiver for communication via a wireless communications network. Thus, if the predictive scheduling model 140 is physically separate from other components of the system 100 shown in FIG. 1, the communication interfaces 28 can enable wireless communications between the predictive scheduling model 140 and these separate components.

Because robotic tools are typically shared in several procedures during the day, the predictive scheduling model 140 may predict surgery time for each procedure, identify tools needed for procedures and optimize their availability and use for each procedure, taking into account hospital and surgeon preferences for scheduling surgeries that can be completed in a day and compiling a surgical calendar based on the information. Additionally, the predictive scheduling model 140 may identify future efficiencies of cost and time, and can optimally determine when to purchase additional tools, and the types of additional tools to purchase. Cost features, particularly consumable costs such as cost to complete schedule for that day and cost per procedure may additionally be determined.

Sterilization and cleaning time may be accounted for in the predictive scheduling model 140. For example, surgeries can be scheduled and staggered. The phases of surgery may be staggered so that tools are available at optimal times. As an example, a tool may be necessary for the first phase of a certain surgery, and the predictive scheduling model 140 may determine that the first phase will last 30 minutes. The sterilization and cleaning time may be 60 minutes for the tool. As such, the predictive schedule model 140 may output a surgical schedule with a first surgery that requires the tool to be available at a given time, e.g., 12:00 PM, and a second surgery requiring the tool to begin 90 minutes after the first surgery, or at 1:30 PM, to optimize use of the tool.

Furthermore, inventory control and scheduling could include identifying relationships between equipment, such as surgical robots and guidance carts, or particular tools and tool trays. For instance, a surgical robot may require a guidance cart or a particular tool may require use with a particular tray. As such, the inventory control and optimization accounts for these relationships, and ensures that all items necessary for use in conjunction are available together, and thus use of these tools is optimized.

The predictive scheduling model 140 may be updated continuously or at given time intervals, e.g., hourly, nightly, weekly, etc., to account for items that may impact scheduling on a given day, such as illness of staff, staff vacations, etc.

The schematic electronic data processing system 100 includes a plurality of outputs, including facility outputs 150, for example, to a hospital or other medical facility where surgeries are performed, and medical device provider outputs 160, for example, to a provider of medical equipment and tools, such as surgical robots and guidance carts. The outputs may include, in addition to a surgical schedule, inventory requirements per day, predicted rental inventory requirements to account for inventory necessary but not yet purchased by the facility, and predicted costs per day. Further outputs include estimated cost savings associated with specific actions, e.g., schedule changes, inventory purchases, etc., and specific cost saving actions (e.g., add one robot or remove one robot for the day) and long-term determinations such as a determination of the return on investment for purchasing an additional tool or surgical robot, e.g., a cost-benefit analysis of purchasing an additional tool or robot.

In some instances, the facility outputs 150 include additional resource recommendations and associated return on investment (ROI) 151. The additional resource recommendations may include suggestions in terms of additional infrastructure, personnel, and/or equipment that may improve facility throughput, reimbursement, or other facility objectives. Based on these recommendations, the output also includes a measure in time of return cost through additional procedures, reduced overhead time, and other savings.

In some instances, the facility outputs 150 include additional equipment recommendations and associated return on investment (ROI) 152. The additional resource recommendations may include suggestions in terms of additional equipment that may be provided by a medical device provider to enable additional procedures, reduce overhead, or other facility objectives. For instance, there may be recommendations for new systems, or additional subcomponents of systems, such as a surgical robot and a guidance cart, just a cart, or additional tool trays. Based on these recommendations, the output also includes an estimated measure of time to recoup the cost of the recommended item, e.g. through additional procedures, reduced overhead time, and other savings.

In some instances, the facility outputs 150 include schedule changes and return on investment (ROI) 153. For instance, changes to a current schedule or resource utilization to enable more effective use of available resources. The system also determines reductions in cost, or better alignment to facility objectives as a result of the schedule changes to determine ROI. In some instances, the facility outputs 150 include a robotic device and enabling technician schedule 154 as part of the schedule changes, to help determine when and how to use non-facility personnel, such as those associated with the medical device provider, e.g., surgical robot technicians.

The facility outputs 150 may be transmitted to a facility scheduling system 170. The results of the facility scheduling system, e.g., an updated facility schedule, may be used as an input to the predictive scheduling model to provide an iteratively updated facility schedule.

The medical device provider outputs 160 may be sent to a medical device provider, e.g., a provider of surgical robots and guidance carts that contracts with the facility, to better serve the facility's needs. For example, in some instances, a medical device provider output 160 includes case specific equipment needs 161, which includes a facility's preferences in terms of reimbursement, and/or case specific resource needs 162, which includes the number of cases performed at the facility per a given time period, e.g., per day, week, month, etc.

As such, the outputs are used to generate a surgical equipment use schedule and operating schedule based on various parameters, and to help facilities, e.g., hospitals, manage capital equipment across one or more facilities. This may include predicting which patients would benefit most from a particular technology type (i.e., from a robot) and assign the procedures for those patients to be done robotically. If more robotic procedures are suggested than the facility has the equipment and scheduling capacity for, the output schedule may also suggest which procedures to perform robotically and which to do manually.

A user may input the pieces of equipment they have and a surgical schedule, and the system could output a recommended equipment schedule. Surgical time prediction algorithms (using CT image data or other patient specific information) may be included as an input to determine the equipment schedule. The system then evaluates when a surgical robot is needed in a procedure, not necessarily the total procedure time, and determine when the robot can be moved once the robot's task is completed in a procedure. The system may evaluate the time needed per each tool. A communication system may be used, such as the Vocera communication system, to communicate the equipment schedule throughout a facility in real-time, and may be updated when equipment needs to be moved. The communication system may include procedure workflow software that self-reports its current status. The system may account for equipment cleaning time, and distance between currently location and a next location the equipment is needed (e.g. the distance the equipment needs to be moved from its current location to its next procedure location in the facility).

Referring to FIG. 2, the electronic data processing system 100 may include one or more preoperative measurement systems 200 which collect and/or output preoperative data 1000 about the instant (current) patient and/or prior patients (e.g., similar prior patients). The predicted surgical case time model 121 may receive and analyze the preoperative data 1000 and generate one or more outputs or determinations 2000, which may be output to one or more output systems 250.

The preoperative measurement systems 200 may include an imaging device 210, electronic devices storing electronic medical records (EMR) 220; patient, practitioner, and/or user interfaces or applications 230 (such as on tablets, computers, or other mobile devices); and a robotic and/or automated data system or platform 240 (e.g., MAKO Robot System or platform, MakoSuite, etc.), which may have a robotic device 242. The electronic data processing system 100 may collect current imaging data 1010 via the imaging device 210 and supplemental or additional information (e.g., patient data and medical history 1020, planned procedure data 1030, surgeon and/or staff data 1040, and/or prior procedure data 1050) via EMR 220, interfaces 230, sensors and/or electronic medical devices, and/or robotic platform 240. Each of the devices in the preoperative measurement systems 200 (the imaging device 210, EMR 220, user interfaces or applications 230, sensors and/or electronic medical devices, and robotic platform 240) may include one or more communication modules (e.g., WiFi modules, BlueTooth modules, etc.) configured to transmit preoperative data 1000 to each other, to the predicted surgical case time model 121, and/or to the one or more output systems 250.

The imaging device 210 may be configured to collect or acquire one or more images, videos, or scans of a patient's internal anatomy, such as bones, ligaments, soft tissues, brain tissue, etc. to provide imaging data 1010, which will be described in more detail later. The imaging device 210 may include a computed tomography (CT) scanner, a magnetic resonance imaging (MRI) machine, an x-ray machine, a radiography system, an ultrasound system, a thermography system, a tactile imaging system, an elastography, nuclear medicine functional imaging system, a positron emission tomography (PET) system, a single-photon emission computer tomography (SPECT) system, a camera, etc. The collected images, videos, or scans may be transmitted, automatically or manually, to the predicted surgical case time model 121. In some examples, a user may select specific images from a plurality of images taken with an imaging device 210 to be transmitted to the predicted surgical case time model 121.

The electronic data processing system 100 may use previously collected data from EMR 220, which may include patient data and medical history 1020 in the form of past practitioner assessments, medical records, past patient reported data, past imaging procedures, treatments, etc. For example, EMR 220 may contain data on demographics, medical history, biometrics, past procedures, general observations about the patient (e.g., mental health), lifestyle information, data from physical therapy, etc. Patient data and medical history 1020 will be described in more detail later.

The electronic data processing system 100 may also collect present or current (e.g., in real time) patient data via patient, practitioner, and/or user interfaces or applications 230. These user interfaces 230 may be implemented on mobile applications and/or patient management websites or interfaces, such as OrthologIQ^{®}. User interfaces 230 may present questionnaires, surveys, or other prompts for practitioners or patients to enter assessments (e.g., throughout a prehabilitation program prior to a procedure), observed psychosocial information and/or readiness for surgery, comments, etc. for additional patient data 1020. Patients may also enter psychosocial information such as perceived or evaluated pain, stress level, anxiety level, feelings, and other patient reported outcome measures (PROMS) into these user interfaces 230. Patients and/or practitioners may report lifestyle information via user interfaces 230. User interfaces 230 may also collect clinical data such as planned procedure 1030 data and planned surgeon and/or staff data 1040 described in more detail later. These user interfaces 230 may be executed on and/or combined with other devices disclosed herein (e.g., with robotic platform 240).

The electronic data processing system 100 may collect prior procedure data 1050 from prior patients and/or other real-time data or observations (e.g., observed patient data 1020) via robotic platform 240. The robotic platform 240 may include one or more robotic devices (e.g., surgical robot 242), computers, databases, etc. used in prior procedures with different patients. The surgical robot 242 may have assisted with, via automated movement, surgeon assisted movement, and/or sensing, a prior procedure and may be implemented as or include one or more automated or robotic surgical tools, robotic surgical or Computerized Numerical Control (CNC) robots, surgical haptic robots, surgical tele-operative robots, surgical hand-held robots, or any other surgical robot.

Although the preoperative measurement system(s) 100 is described in connection with imaging device 1010, EMR 220, user interfaces 230, and robotic platform 240, other devices may be used preoperatively to collect preoperative data 1000. For example, mobile devices such as cell phones and/or smart watches may include various sensors (e.g., gyroscopes, accelerometers, temperature sensors, optical or light sensors, magnetometer, compass, global positioning systems (GPS) etc.) to collect patient data 1020 such as location data, sleep patterns, movement data, heart rate data, lifestyle data, activity data, etc. As another example, wearable sensors, heart rate monitors, motion sensors, external cameras, etc. having various sensors (e.g., cameras, optical light sensors, barometers, GPS, accelerometers, temperature sensors, pressure sensors, magnetometer or compass, MEMs devices, inclinometers, acoustical ranging, etc.) may be used during physical therapy or a prehabilitation program to collect information on patient kinematics, alignment, movement, fitness, heart rate, electrocardiogram data, breathing rate, temperature, oxygenation, sleep patterns, activity frequency and intensity, sweat, perspiration, air circulation, stress, step pressure or push-off power, balance, heel strike, gait, fall risk, frailty, overall function, etc. Other types of systems or devices that may be used in the preoperative measurement system 10 may include electromyography or EMG systems or devices, motion capture (mocap) systems, sensors using machine vision (MV) technology, virtual reality (VR) or augmented reality (AR) systems, etc.

The preoperative data 1000 may be data collected, received, and/or stored prior to an initiation of a medical treatment plan or medical procedure. As shown by the arrows in FIG. 2, the preoperative data 1000 may be collected using the preoperative measurement systems 200, from memory system 20 (e.g., cloud storage system) of the predicted surgical case time model 121, and from output systems 250 (e.g., from a prior procedure) for one or more continuous feedback loops. Some of the preoperative data 1000 may be directly sensed via one or more devices (e.g., wearable motion sensors or mobile devices) or may be manually entered by a medical professional, patient, or other party. Other preoperative data 1000 may be determined (e.g., by predicted surgical case time model 121) based on directly sensed information, input information, and/or stored information from prior medical procedures.

As previously described, the preoperative data 1000 may include imaging data 1010, patient data and/or medical history 1020, information on a planned procedure 1030, surgeon data 1040, and prior procedure data 1050.

The imaging data 1010 may include morphology and/or anthropometrics (e.g., physical dimensions of internal organs, bones, etc.), fractures, slope or angular data, tibial slope, posterior tibial slope or PTS, bone density, (e.g., bone mineral or bone marrow density, bone softness or hardness, or bone impact), etc. Bone density may be determined separately using the predicted surgical case time model 121, as described in more detail later, and/or may be collected or supplemented using, for example, indent tests or a microindentation tool. Imaging data may not be limited to strictly bone data and may be inclusive of other internal imaging data, such as of cartilage, soft tissue, or ligaments, and non-orthopedic data such as tumor position, tumor size, disease state, and inflammation, among other parameters.

The imaging data 1010 may be in a form of raw images, videos, or scans collected by the imaging device 210 and to be analyzed by the predicted surgical case time model 121. The images or scans may illustrate or indicate bone, cartilage, or soft tissue positions or alignment, composition or density, fractures or tears, bone landmarks (e.g., condyle surface, head or epiphysis, neck or metaphysis, body or diaphysis, articular surface, epicondyle, lateral epicondyle, medial epicondyle, process, protuberance, tubercle vs tuberosity, tibial tubercle, trochanter, spine, linea or line, facet, crests and ridges, foramen and fissure, meatus, fossa and fovea, incisure and sulcus, and sinus), geometry (e.g., diameters, slopes, angles) and/or other anatomical geometry data such as deformities or flare (e.g., coronal plane deformity, sagittal plane deformity, lateral femoral metaphyseal flare, or medial femoral metaphyseal flare). Such geometry is not limited to overall geometry and may include relative dimensions (e.g., lengths or thicknesses of a tibia or femur). The imaging data 1010 may indicate or be used to determine osteophyte size, volume, or positions; bone loss; joint space; B-score; bone quality/density; skin-to-bone ratio; bone loss; hardware detection; anterior-posterior (AP) and medial-lateral (ML) distal femur size, and/or joint angles. Analysis and/or calculations that may be derived from the images or scans will be described in more detail later when describing the predicted surgical case time model 121.

In addition to raw images, imaging data 1010 may include intermediate and/or related imaging data 1010 to be used by the predicted surgical case time model 121 to calculate outputs 2000. Such intermediate imaging data 1010 may include density or composition charts or graphs; quantified data indicating relative positions, dimensions, etc.; and/or processed image data indicating specifically detected attributes, such as a probability of a certain patient condition. One or more algorithms 90 of the procedure prediction system 121 may determine or calculate this intermediate imaging data 1010 in determining outputs 2000, or alternatively or additionally thereto, the imaging device 210 may include one or more processors configured to calculate or quantify, based on the raw images, videos, or scans, at least some of the intermediate imaging data 1010. Intermediate imaging data 1010 may include information relating to, indicating, and/or quantifying aspects of the raw images, charts, etc.

Patient data and medical history 1020 may include information about the instant patient on identity (e.g., name or birthdate), demographics (e.g., patient age, gender, height, weight, nationality, body mass index (BMI), etc.), lifestyle (e.g., smoking habits, exercise habits, drinking habits, eating habits, fitness, activity level, frequency of climbing activities such as up and down stairs, frequency of sit-to-stand movements or bending movements such as when entering and exiting a vehicle, steps per day, activities of daily living or ADLs performed, etc.), medical history (e.g., allergies, disease progressions, addictions, prior medication use, prior drug use, prior infections, frailties, comorbidities, prior surgeries or treatment, prior injuries, prior pregnancies, utilization of orthotics, braces, prosthetics, or other medical devices, etc.), assessments and/or evaluations (e.g., laboratory tests and/or bloodwork, American Society of Anesthesiology or ASA score and/or fitness for surgery or aesthesia) electromyography data (muscle response or electrical activity in response to a nerve's stimulation), psychosocial information (e.g., perceived pain, stress level, anxiety level, mental health status, PROMS (e.g., knee injury and osteoarthritis outcome score or KOOS, hip disability and osteoarthritis outcome score or HOOS, pain virtual analog scale or VAS, PROMIS Global 10 or PROMIS-10, EQ-5D, a mental component summary, satisfaction or expectation information, etc.), past biometrics (e.g., heart rate or heat rate variability, electrocardiogram data, breathing rate, temperature (e.g., internal or skin temperature), fingerprints, DNA, etc.), past kinematics or alignment data, past imaging data, data from prehabilitation programs or physical therapy (e.g., average load bearing time) etc. Medical history 1020 may include prior clinical or hospital visit information, including encounter types, dates of admission, hospital-reported comorbidity data such as Elixhauser and/or Charlson scores or selected comorbidities (e.g., ICD-10 POA), prior anesthesia taken and/or reactions, etc. This list, however, is not exhaustive and preoperative data 1000 may include other patient specific information, clinical information, and/or surgeon or practitioner specific information (e.g., experience level).

Patient data 1020 may come from EMR 220, user interfaces 230, from memory system 20, and/or from robotic platform 240, but aspects disclosed herein are not limited to a collection of the patient data 1020. For example, other types of patient data 1020 or additional data may include data on activity level; kinematics; muscle function or capability; range of motion data; strength measurements and/or force measurements push-off power, force, or acceleration; a power, force, or acceleration at a toe during walking; angular range or axes of joint motion or joint range of motion; flexion or extension data, including step data (e.g., measured by a pedometer), gait data or assessments; fall risk data; balancing data; joint stiffness or laxity data; postural sway data; data from tests conducted in a clinic or remotely; etc.

Information on a planned procedure 1030 may include logistical information about the procedure and substantive information about the procedure. Logistical planned procedure 1030 information may include information about a planned site of the procedure such as a hospital, ambulatory surgery center (ASC), or an operating room; a type of procedure or surgery to be performed (e.g., total or partial knee arthroplasty or replacement, total or partial hip arthroplasty or replacement, spine surgery, patella resurfacing, etc.); scheduling or booking information such as a date or time of the procedure or surgery, planning or setup time, registration time, and/or bone preparation time, or other robotic task completion time; a disease or infection state of the surgeon; a name of the primary surgeon or doctor who plans to perform the procedure; equipment or tools required for the procedure; medication or other substances required (e.g., anesthesia type) for the procedure; insurance type or billing information; consent and waiver information; etc. Substantive planned procedure 1030 information may include a surgeon's surgical or other procedure or treatment plan, including planned steps or instructions on incisions, a side of the patient's body to operate on (e.g., left or right) and/or laterality information, bone cuts or resection depths, implant design, type, and/or size, implant alignment, fixation or tool information (e.g., implants, rods, plates, screws, wires, nails, bearings used), cementing versus cementless techniques or implants, final or desired alignment, pose or orientation information (e.g., capture gap values for flexion or extension, gap space or width between two or more bones, joint alignment), planning time, gap balancing time, extended haptic boundary usage, etc. This initial planned procedure 1030 information may be manually prepared or input by a surgeon and/or previously prepared or determined using one or more algorithms.

Surgeon data 1040 may include information about a surgeon or other staff planned to perform the planned procedure 1030. Surgeon data 1040 may include identity (e.g., name), experience level, fitness level, height and/or weight, etc. Surgeon data 1040 may include number of surgeries scheduled for a particular day, number of complicated surgeries scheduled on the day of a planned procedure, average surgery time, surgeon equipment preferences, etc.

Prior procedure data 1050 may include information about prior procedures performed on a same or prior patient. Such information may include the same type of information as in planned procedure data 1030 (e.g., instructions or steps of a procedure, bone cuts, implant design, implant alignment, etc.) along with outcome and/or result information, which may include both immediate results and long-term results, complications after surgery, length of stay in a hospital, revision surgery data, rehabilitation data, patient motion and/or movement data, etc. Prior procedure data 1050 may include information about prior procedures of prior patients sharing at least one same or similar characteristic (e.g., demographically, biometrically, disease state, etc.) as the instant patient.

Preoperative data 1000 may include any other additional or supplemental information stored in memory system 20, which may also include known data and/or data from third parties, such as data from the Knee Society Clinical Rating System (KSS) or data from the Western Ontario and McMaster Universities Osteoarthritis Index (WOMAC).

The predicted surgical case time model 121 may be an artificial intelligence (AI) and/or machine learning system that is "trained" or that may learn and refine patterns between preoperative data 1000, outputs 2000, and actual results 12 (FIG. 1) to make determinations. The predicted surgical case time model 121 may be implemented using one or more computing platforms, such as platforms including one or more computer systems and/or electronic cloud processing systems. Examples of one or more computing platforms may include, but are not limited to, smartphones, wearable devices, tablets, laptop computers, desktop computers, Internet of Things (loT) device, remote server/cloud based computing devices, or other mobile or stationary devices. The predicted surgical case time model 121 may also include one or more hosts or servers connected to a networked environment through wireless or wired connections. Remote platforms may be implemented in or function as base stations (which may also be referred to as Node Bs or evolved Node Bs (eNBs)). Remote platforms may also include web servers, mail servers, application servers, etc.

The predicted surgical case time model 121 may include one or more communication modules (e.g., WiFi or Bluetooth modules) configured to communicate with preoperative measurement systems 200, output system 200, and/or other third-party devices, etc. For example, such communication modules may include an Ethernet card and/or port for sending and receiving data via an Ethernet-based communications link or network, or a Wi-Fi transceiver for communication via a wireless communications network. Such communication modules may include wired or wireless interfaces (e.g., jacks, antennas, transmitters, receivers, transceivers, wire terminals, etc.) for conducting data communications with external sources via a direct connection or a network connection (e.g., an Internet connection, a LAN, WAN, or WLAN connection, LTE, 4G, 5G, Bluetooth, near field communication (NFC), radio frequency identifier (RFID), ultrawideband (UWB), etc.). Such communication modules may include a radio interface including filters, converters (for example, digital-to-analog converters and the like), mappers, a Fast Fourier Transform (FFT) module, and the like, to generate symbols for a transmission via one or more downlinks and to receive symbols (for example, via an uplink).

The predicted surgical case time model 121 may further include the memory system 20 and a processing circuit 40. The memory system 20 may have one or more memories or storages configured to store or maintain the preoperative data 1000, outputs 2000, and stored data 30 from prior patients and/or prior procedures. The preoperative data 1000 and outputs 2000 of an instant procedure may also become stored data 50. Although certain information is described in this specification as being preoperative data 1000 or outputs 2000, due to continuous feedback loops of data (which may be anchored by memory system 20), the preoperative data 1000 described herein may alternatively be determinations or outputs 2000, and the determined outputs 2000 described herein may also be used as inputs into the predicted surgical case time model 121. For example, some preoperative data 1000 may be directly sensed or otherwise received, and other preoperative data 1000 may be determined, processed, or output based on other preoperative data 1000. Although the memory system 20 is illustrated close to processing circuit 40, memory system 20 may include memories or storages implemented on separate circuits, housings, devices, and/or computing platforms and in communication with predicted surgical case time model 121, such as cloud storage systems and other remote electronic storage systems.

The memory system 20 may include one or more external or internal devices (random access memory or RAM, read only memory or ROM, Flash-memory, hard disk storage or HDD, solid state devices or SSD, static storage such as a magnetic or optical disk, other types of non-transitory machine or computer readable media, etc.) configured to store data and/or computer readable code and/or instructions that completes, executes, or facilitates various processes or instructions described herein. The memory system 20 may include volatile memory or non-volatile memory (e.g., semiconductor-based memory device, a magnetic memory device and system, an optical memory device and system, fixed memory, or removable memory). The memory system 20 may include database components, object code components, script components, or any other type of information structure to support the various activities described herein. In some aspects, the memory system 20 may be communicably connected to the processing circuit 40 and may include computer code to execute one or more processes described herein. The memory system 20 may contain a variety of modules, each capable of storing data and/or computer code related to specific types of functions.

The processing circuit 40 may include a processor 42 configured to execute or perform one or more algorithms 90 based on received data, which may include the preoperative data 1000 and/or any data in the memory system 20 to determine the outputs 2000. The preoperative data 1000 may be received via manual input, retrieved from the memory system 20, and/or received direction from the preoperative measurement systems 200. The processor 42 may be configured to determine patterns based on the received data.

The processor 42 may be implemented as a general purpose processor or computer, special purpose computer or processor, microprocessor, digital signal processor (DSPs), an application specific integrated circuit (ASIC), one or more field programmable gate arrays (FPGAs), a group of processing components, processor based on a multi-core processor architecture, or other suitable electronic processing components. The processor 42 may be configured to perform machine readable instructions, which may include one or more modules implemented as one or more functional logic, hardware logic, electronic circuitry, software modules, etc. In some cases, the processor 42 may be remote from one or more of the computing platforms comprising the predicted surgical case time model 121. The processor 42 may be configured to perform one or more functions associated with the predicted surgical case time model 121, such as precoding of antenna gain/phase parameters, encoding and decoding of individual bits forming a communication message, formatting of information, and overall control of one or more computing platforms comprising the predicted surgical case time model 121, including processes related to management of communication resources and/or communication modules.

In some aspects, the processing circuit 50 and/or memory system 20 may contain several modules related to medical procedures, such as an input module, an analysis module, and an output module. The predicted surgical case time model 121 need not be contained in a single housing. Rather, components of the predicted surgical case time model 121 may be located in various different locations or even in a remote location. Components of the predicted surgical case time model 121, including components of the processing circuit 40 and the memory system 20, may be located, for example, in components of different computers, robotic systems, devices, etc. used in surgical procedures.

The predicted surgical case time model 121 may use the one or more algorithms 90 to make intermediate determinations and to determine the one or more outputs 2000. The one or more algorithms 90 may be configured to determine or glean data from the preoperative data 1000, including the imaging data 1010. For example, the one or more algorithms 90 may be configured for bone recognition, soft tissue recognition, and/or to make determinations related to the intermediate imaging data 1010 previously described.

The one or more algorithms 90 may be machine learning algorithms that are trained using, for example, linear regression, random forest regression, CatBoost regression, etc. The one or more algorithms 90 may be continuously modified and/or refined based on actual outcomes and/or results 12 (FIG. 1). The one or more algorithms 90 may be configured to use segmenting techniques and/or thresholding techniques on received images, videos, and/or scans of the imaging data 1010 to determine the previously described intermediate imaging data 1010 and/or the one or more outputs 2000. For example, the one or more algorithms 90 may be configured to segment an image (e.g., a CT scan), threshold soft tissue, generate a .txt comparisons of certain identified bones or tissues (e.g., tibia and femur), and run code to extract values (e.g., PPT or PTT) and populate a database. The one or more algorithms 90 may be configured to automate data extraction and/or collection upon receiving an image from the imaging device 210.

As will be described in more detail in connection with the one or more algorithms 90 and the output system 200, the one or more outputs 2000 may include a predicted procedure time or duration 2010, a procedure plan 2020, an operating room layout 2030, an operating room schedule 2040, assigned or designated staff 2050, recommended surgeon ergonomics 2070, and predicted outcomes 2080 of the procedure. The predicted procedure time 2010 may be a total time or duration of a procedure (e.g., as outlined in the procedure plan 2020), and may further include a time or duration of steps or processes of the procedure, and/or groups of steps or processes of the procedure. In some examples, the predicted procedure time 2010 may be a predicted time to complete a portion of a procedure. The procedure plan 2020, the operating room layout 2030, the operating room schedule 2040, the assigned staff 2050, the recommended surgeon ergonomics 2070, and the predicted outcomes 2080 may be determined based on the determined predicted procedure time 2010. The predicted outcomes 2080 may include a predicted perceived pain level for the patient, a predicted stress level, anxiety level, and/or mental health status of the patient, a predicted cartilage loss, a predicted risk of infection, a rating of a case difficulty, etc. The predicted outcomes 2080 may also include predictions and/or risks if, during the procedure, a time exceeds (or alternatively, is less than) the predicted procedure time 2010 (for example, how a risk of complication and/or a risk of infection may increase based on the procedure taking longer than the predicted procedure time 2010).

The one or more algorithms 90 may include a joint-space width algorithm 50, an osteophyte volume algorithm 60, a B-score algorithm 70, and an alignment/deformity algorithm 80. Alternatively, one or more of these algorithms may be combined. For example, the joint-space width algorithm 50, the osteophyte volume algorithm 60, the B-score algorithm 70, and the alignment/deformity algorithm 80 may be combined in a single or master algorithm. Each of the joint-space width algorithm 50, the osteophyte volume algorithm 60, the B-score algorithm 70, and the alignment/deformity algorithm 80 may be configured to use not only preoperative data 1000 as input but also determinations and/or outputs 2000 from each other. Each of the one or more algorithms 90 (the joint-space width algorithm 50, the osteophyte volume algorithm 60, the B-score algorithm 70, and the alignment/deformity algorithm 80) may be configured to use image processing techniques to recognize or detect bones, tissues, bone landmarks, etc. and calculate or predict dimensions and/or positions thereof. The one or more algorithms are not limited to determinations relating to joint-space width, osteophyte volume, B-score, and alignment/deformity, and may include and/or be configured to make other procedural determinations, such as those relating to joint laxity or stiffness, discharge time or length of stay time, frailty, fall risk, balancing assessments, patient readiness, etc.

A joint space width (JSW) may be a distance between two or more bones at a joint. The joint-space width algorithm 50 may be configured to determine one or more JSW parameters from the preoperative data 1000 (e.g., imaging data 1010) relating to a joint space width in one or more target joints. The one or more JSW parameters may include joint space widths at predetermined locations, joint space widths across different directions (e.g., medial JSW or lateral JSW), average or mean joint space width (e.g., mean three-dimensional or 3D joint space width), changing joint-space (e.g., joint space narrowing), an average or mean joint space narrowing (e.g., mean 3D joint space narrowing), impingement data, impingement angles, impingement data based on a predicted or determined implant, etc. The joint-space width algorithm 50 may detect and/or reference a plurality (e.g., hundreds) of bone landmarks to determine joint space widths at various positions.

The joint-space width algorithm 50 may assess one or more of these JSW parameters at various anatomical compartments (e.g., anterior lateral, anterior medial, central lateral, central medial, posterior lateral, posterior medial) of one or more bones (e.g., tibia and femur). The joint space width algorithm 50 may also be configured to predict joint spaces based on loadbearing and/or unloaded conditions using other preoperative data 1000, such as kinematics data or activity level data.

The joint space width algorithm 50 may, based on supplemental patient data 1030, determine whether a joint space width is decreasing or narrowing (and/or increasing or widening) based on a comparison of previously measured joint space widths and/or based on a comparison of imaging data from previous image acquisitions. The joint space width algorithm 50 may also determine cartilage thickness or determine predict a cartilage loss during the procedure (e.g., by using a Z-score or other statistical measure).

For example, The joint-space width algorithm 50 may also be used to determine scores or values in a plurality (e.g., four) of anatomical compartments (e.g., knee joint) based on joint-space width or cartilage loss, and determine a composite score or C-score based on the determined scores of each of the compartments. The scores for each compartment and/or the C-score may also be based on patient data 1020, such as gender, as males and females on average have different cartilage widths. The joint-space width algorithm 50 may determine or select a compartment among the plurality of compartments that should be resurfaced during the procedure, and determine that the procedure plan 2020 should include one or more steps directed to resurfacing the selected compartment. The joint-space width algorithm 50 may determine cartilage thickness or loss based on a determined C-score, and may consider patient data 1020 (e.g., gender). The joint-space width algorithm 50 may convert a joint-space width (e.g., in mm) to a Z-score or other score. A Z-score may describe a relationship between a particular value (e.g., joint-space width) with a mean or average of a group of values. For example, a Z-score may be measured in terms of standard deviations from the mean such that Z-score of 0 may indicate a value that is identical to the mean score. In some examples, the joint-space width algorithm 50 may determine patient data 1020, such as gender, based on the determined JSW parameters (e.g., C-score or Z-score). In some examples, the joint-space width algorithm 50 may determine whether the procedure plan 2020 should include a total or partial arthroplasty (e.g., a total or partial knee arthroplasty).

Based on the determined JSW parameters, the joint-space width algorithm 50 and/or the one or more algorithms 90 collectively may be used to determine one or more of the outputs 2000. In some examples, the joint-space width algorithm 50 may determine and/or predict (or be used to determine and/or predict) a procedure time or duration 2010 to execute a procedure plan 2020. For example, the joint-space width algorithm 50 may determine that a joint space width of a patient is outside of a predetermined range, is narrowing over time and/or is smaller than a first predetermined threshold, or is widening over time and/or is greater than a second predetermined threshold. The predicted surgical case time model 121 may, based at least in part on these determinations by the JSW algorithm 50, predict a longer or shorter procedure time 2010 (for example, based on a function where the predicted time is inversely proportional or proportional to the joint space width, and/or based on a step-wise increase based on predetermined thresholds, etc.) Other factors (e.g., from patient data 1020) may change the analysis and/or relationship such that the predicted surgical case time model 121 and/or the osteophyte joint-space width algorithm 50 may determine certain relationships between higher or lower JSW parameters combined with certain patient data 1020. Details of the other outputs 2000 will be described in more detail hereinafter in connection with all of the algorithms 90.

An osteophyte may be a bone spur that develops on a bone. Osteophyte volume may refer to a total volume of osteophytes on a bone or a specific portion of a bone. The osteophyte volume algorithm 60 may be configured to detect or recognize one or more osteophytes at a target bone, joint, or portion of a bone, and determine or calculate one or more osteophyte parameters from the preoperative data 1000 (e.g., imaging data 1010) relating to osteophyte detection or osteophyte dimensions (e.g., volume) of one or more detected osteophytes in one or more target joints). The one or more osteophyte parameters may include an osteophyte location, an osteophyte number, osteophyte volumes at predetermined locations, osteophyte areas across different directions (e.g., medial or lateral), an average or mean osteophyte volume, changing or progressing osteophyte volume, impingement data, impingement angles, impingement data based on a predicted or determined implant, etc. The osteophyte volume algorithm 60 may assess one or more of these osteophyte parameters at one or more bones (e.g., femur or tibia) and/or various anatomical compartments (e.g., anterior lateral, anterior medial, central lateral, central medial, posterior lateral, posterior medial) of one or more bones (e.g., tibia and femur). The osteophyte volume algorithm 60 may also be configured to predict osteophyte volume or progression based on other preoperative data 1000, such as kinematics data or activity level data.

The osteophyte volume algorithm 60 may, based on supplemental patient data 1030, determine whether osteophyte volume (e.g., total osteophyte volume or an osteophyte volume of a specific region or osteophyte) is increasing or decreasing based on a comparison of previously measured osteophyte volumes and/or based on a comparison of imaging data from previous image acquisitions. The osteophyte volume algorithm 60 may further determine, predict, or diagnose a disease state or a disease progression (e.g., osteoarthritis or OA) based on the determined osteophyte parameters.

Based on the determined osteophyte parameters, the osteophyte volume algorithm 60 and/or the one or more algorithms 90 collectively may be used to determine one or more of the outputs 2000. The osteophyte volume algorithm 60 may determine and/or predict (or be used to determine and/or predict) the procedure time 2010 to execute the procedure plan 2020. For example, the osteophyte volume algorithm 60 may determine that an osteophyte volume of a patient is progressing over time and/or is larger than a predetermined threshold, and predict a longer procedure time 2010 (for example, based on a function where the predicted time is proportional to the osteophyte volume, and/or based on a step-wise increase based on predetermined thresholds, etc.) However, a higher osteophyte volume may not necessarily result in a longer procedure time 2010, as other factors (e.g., from patient data 1020) may change the analysis such that the predicted surgical case time model 121 and/or the osteophyte volume algorithm 60 may determine different relationships between higher or lower osteophyte volumes combined with certain patient data 1020 (e.g., shorter procedure time 2010 based on a higher osteophyte volume and certain patient data 1020). Details of the other outputs 2000 will be described in more detail hereinafter.

B-score may be a type of score or scoring system based on and/or quantifying a shape of a femur or knee joint. B-score may be a holistic, average, or overall score indicating an overall assessment of the femur and/or the knee, but knees having different specific complications or deformities may result in similar B-scores. The B-score may be based on how the shape of the femur compares to knee shapes of those with OA and knee shapes of those who do not have OA, and may be determined using, for example, statistical shape modelling (SSM) or other processes. B-score may be a continuous, quantitative variable, which may be used to quantify overall amount of OA damage in the knee, and also to measure progression in longitudinal studies.

As OA progresses, each bone may exhibit a characteristic shape change, involving osteophyte growth around cartilage plates, and a spreading and flattening of a subchondral bone. A femur shape change may increase regardless of an anatomical compartment affected, and may be more sensitive to change than the tibia and patella. The B-score may represent a distance along the "OA" shape change in the femur bone.

In some examples, a B-score may be recorded as a z-score, similar to a T-score in osteoporosis, which may represent units of standard deviation (SD) of a healthy population, with 0 defined as ae mean of a healthy population. Values of -2 to +2 may represent a healthy population, whereas values above +2 may fall beyond the healthy population.

The B-score algorithm 70 may be configured to determine a B-score from imaging data 1080 containing images and/or related data of a knee and/or femur. The B-score may be based in part on, or correlate to, OA progression, where a B-score of 0 may correlate to and/or indicate a mean femur shape of those who do not have OA. Further details of how B-score is calculated may be found in "Machine-learning, MRI bone shape and important clinical outcomes in osteoarthritis: data from the Osteoarthritis Initiative" by Michael A. Bowes, Katherine Kacena, Oras A. Alabas, Alan D. Brett, Bright Dube, Neil Bodick, Philip G Conaghan published November 13, 2020, which is incorporated reference herein in its entity. Aspects disclosed herein are not limited to such a B-score, however. For example, the B-Score algorithm 70 may additionally and/or alternatively calculate other scores or quantifications of other bone shapes based on how they compare to bone shapes of those having a particular disease.

The B-score algorithm 70 may be configured to detect or recognize one or more target bones or joint (e.g., femur), detect or recognize a shape of the target bone or joint, and/or determine or calculate one or more shape score parameters from the preoperative data 1000 (e.g., imaging data 1010) relating to the shape of the target bone and/or how that shape compares with prior patients having a particular disease. For ease of description, an example where the B-score algorithm 70 calculates one or more B-score parameters in connection to knee and/or femur will be described. The one or more B-score parameters may include B-scores at different times or in different images, an average or mean B-score, and/or a changing or progressing B-score. The B-score algorithm 70 may also be configured to predict a future B-score or B-score progression based on other preoperative data 1000, such as kinematics data or activity level data.

The B-score algorithm 70 may, based on supplemental patient data 1030, determine whether a B-score for a particular femur (e.g., left femur) or both femurs is increasing or decreasing based on a comparison of previously measured B-scores and/or based on a comparison of imaging data from previous image acquisitions. The B-score algorithm 70 may further determine, predict, or diagnose a disease state or a disease progression (e.g., osteoarthritis or OA) based on the determined B-score and/or B-score progression.

Based on the determined B-score, the B-score algorithm 70 and/or the one or more algorithms 90 collectively may be used to determine one or more of the outputs 2000. The B-score algorithm 70 may determine and/or predict (or be used to determine and/or predict) the procedure time 2010 to execute the procedure plan 2020. For example, the B-score algorithm 70 may determine that certain patient data 1020 combined with a B-score of a patient is progressing over time and/or is larger than a predetermined threshold, and predict a longer procedure time 2010 (for example, based on a function where the predicted time is proportional to the B-score, and/or based on a step-wise increase based on predetermined thresholds, etc.)

However, a higher B-score may not necessarily result in a longer procedure time 2010. For example, patients belonging to the U.S. population that have a higher B-score may be associated with longer procedure times, while patients belonging to EU populations that have a higher B-score may be associated with shorter procedure times. Thus, the B-score algorithm 70 and/or predicted surgical case time model 121 may determine a longer procedure time 2010 based on a higher B-score and a patient nationality of U.S. and a shorter procedure time 2010 based on a higher B-score and a patient nationality of an EU country. Other factors (e.g., from patient data 1020) may change the analysis and/or relationship such that the predicted surgical case time model 121 and/or the B-score algorithm 70 may determine certain relationships between higher or lower B-scores combined with certain patient data 1020. Details of the other outputs 2000 will be described in more detail hereinafter.

Alignment and/or deformity may refer to how two or more bones are positioned and/or moved as compared to a healthy patient having a healthy alignment at the two or more bones. The alignment/deformity algorithm 80 may be configured to detect or recognize one or more target bones or joints, detect relative positions and/or dimensions of the one or more target bones or joints, and determine or calculate one or more alignment/deformity parameters from the preoperative data 1000 (e.g., imaging data 1010) relating to alignment detection or osteophyte dimensions (e.g., volume) of one or more detected osteophytes in one or more target joints).

The one or more alignment/deformity parameters may include alignment and/or relative position data at certain locations (e.g., joint location), across different directions (e.g., medial or lateral), an average or mean alignment and/or an alignment score, changing or progressing alignment, alignment based on a predicted or determined implant, etc. The alignment/deformity algorithm 80 may assess one or more of these alignment/deformity parameters at one or more bones (e.g., femur or tibia) and/or various anatomical compartments (e.g., anterior lateral, anterior medial, central lateral, central medial, posterior lateral, posterior medial) of one or more bones (e.g., tibia and femur). The alignment/deformity algorithm 80 may also be configured to predict alignment or progression based on other preoperative data 1000, such as kinematics data or activity level data.

The one or more alignment/deformity parameters may include alignment and/or relative positions (e.g., relative to anatomical and/or mechanical axes), such as lower extremity mechanical alignment, lower extremity anatomical alignment, femoral articular surface angle, tibial articular surface angle, mechanical axis alignment strategy, anatomical alignment strategy, natural knee alignment strategy, femoral bowing, varus-valgus deformity and/or angles, tibial bowing, patello-femoral alignment, coronal plane deformity, sagittal plane deformity, extension motion, flexion motion, anterior cruciate ligament (ACL) ligament intact, posterior cruciate ligament (PCL) ligament intact, knee motion and/or range of motion data (e.g., collected with markers appearing in the raw images, videos, or scans) in all three planes during active and passive range of motion in a joint, three dimensional size, quantified data indicating proportions and relationships of joint anatomy in both static and motion, quantified data indicating height of a joint line, metaphyseal flare, medial femoral metaphyseal flare, proximal tibio-fibular joint, coronal tibial diameter, femoral interepicondylar diameter, femoral intermetaphyseal diameter, sagittal tibial diameter, posterior femoral condylar offset-medial and lateral, lateral epicondyle to joint line distance, and/or tibial tubercle to joint line distance. However, aspects disclosed herein are not limited to these alignment parameters.

The one or more alignment/deformity parameters may include data on bone landmarks (e.g., condyle surface, head or epiphysis, neck or metaphysis, body or diaphysis, articular surface, epicondyle, process, protuberance, tubercle vs tuberosity, trochanter, spine, linea or line, facet, crests and ridges, foramen and fissure, meatus, fossa and fovea, incisure and sulcus, and sinus) and/or bone geometry (e.g., diameters, slopes, angles) and other anatomical geometry data. Such geometry is not limited to overall geometry and may include specific lengths or thicknesses (e.g.,, lengths or thicknesses of a tibia or femur). Imaging data 1010 may also include data on soft tissues for ligament insertions and/or be used to determine ligament insertion sites.

The alignment/deformity algorithm 80 may, based on imaging data 1080 and/or supplemental patient data 1020, determine whether a misalignment, deformity, distances between certain bones, and/or angles between different bones is increasing or decreasing based on a comparison of previously measured alignment/deformity parameters and/or based on a comparison of imaging data from previous image acquisitions. The alignment/deformity algorithm 80 may further determine, predict, or diagnose a disease state or a disease progression (e.g., osteoarthritis or OA) based on the determined alignment/deformity parameters.

Based on the determined alignment/deformity parameters, the alignment/deformity algorithm 80 and/or the one or more algorithms 90 collectively may be used to determine one or more of the outputs 2000. The alignment/deformity algorithm 80 may determine and/or predict (or be used to determine and/or predict) the procedure time 2010 to execute the procedure plan 2020. For example, the alignment/deformity algorithm 80 may determine that a deformity of a patient is progressing over time and/or is larger than a predetermined threshold, and predict a longer procedure time 2010 (for example, based on a function where the predicted time is proportional to the osteophyte volume, and/or based on a step-wise increase based on predetermined thresholds, etc.) Other factors (e.g., from patient data 1020) may change the analysis and/or relationship such that the predicted surgical case time model 121 and/or the alignment/deformity algorithm 80 may determine certain relationships between higher or lower alignment/deformity parameters combined with certain patient data 1020. For example, although alignment/deformity algorithm 80 may determine that a deformity of a patient is minor and/or improving, the alignment/deformity algorithm 80 and/or the predicted surgical case time model 121 may determine a longer procedure time 2010 based on a location of the deformity and/or other patient data 1020 (e.g., gender, height, etc.) Details of the other outputs 2000 will be described in more detail hereinafter.

The one or more algorithms 90 may operate simultaneously (or alternatively, at different times throughout the preoperative and intraoperative periods) and exchange inputs and outputs. The one or more algorithms 90 may be configured to determine other scores, values, and/or parameters and are not limited to joint space width, osteophyte volume, B-score, alignment/deformity, and/or a patient readiness score. For example, the one or more algorithms 90 may be configured to determine scores related to bone density (e.g., T-score), joint stiffness or laxity, patient readiness, bone-to-skin ratio, etc. A patient readiness score may preoperatively indicate a patient's independence and/or readiness to undergo a procedure (e.g., surgery) or if further prehabilitation may be needed to enhance a recovery time post-operatively. In addition, the patient readiness score may intraoperatively or postoperatively indicate a patient's readiness to be discharged from a hospital after the procedure. The predicted surgical case time model 121 may be configured to determine a time period (e.g., number of days) for the patient to wait for the procedure and/or determine other scheduling parameters for the procedure.

The one or more algorithms 90 may be configured for bone recognition and may also be configured to detect or determine prepatellar thickness (PPT) and/or pretubercular thickness (PTT), a minimum distance from bone to skin, tissue-to-bone ratio, bone-to-tissue distances or values, and/or bone-to-tissue distances for PPT and/or PTT, bone-to-skin ratio, etc.

The predicted surgical case time model 121 may determine, from the parameters determined from the one or more algorithms 90, the procedure time 2010. For example, the predicted surgical case time model 121 may determine a longer procedure time 2010 based on a narrower (or narrowing) joint space width and/or a wider (or widening) joint space width determined by the joint space width algorithm 50, a larger (or increasing) osteophyte number and/or volume determined by the osteophyte volume algorithm 60, a larger (or increasing) B-score determined by the B-score algorithm 70, a larger (or increasing) deformity and/or misalignment determined by the alignment/deformity algorithm 80, and/or a larger bone-to-tissue ratio, PPT, and/or PTT determined by the one or more algorithms 90, along with certain combinations of patient data 1020. However, these factors are an example of what may yield a longer procedure time 2010, and may be changed based on other factors of combinations based on other inputs 1000, such as those from patient data 1020. The predicted surgical case time model 121 may be configured to determine new relationships based on certain combinations to more accurately determine procedure time 2010. For example, the predicted surgical case time model 121 system may determine over time that although a higher B-score in US patients may result in a longer procedure time 2010, a higher B-score in EU patients may result in a shorter procedure time 2010. Other combinations and/or factors may further change the analysis and/or relationships of all inputs 1000, parameters determined from the algorithms 90, and the outputs 2000 (e.g., procedure time 2010).

PPT and/or PTT may be a distance measurement between a bone and skin determined using images (e.g., CT scans), and may be used as a proxy or alternative to a manually input BMI. In some examples, PPT and/or PTT at a joint (e.g., knee joint) may provide more precise information than BMI, which may be a whole-body measurement. The predicted surgical case time model 121 may determine a longer procedure time 2010 based on a larger bone-to-tissue ratio, PPT, and/or PTT determined by the one or more algorithms 90 and/or a larger BMI (e.g., input and/or determined by the one or more algorithms 90), as practitioners may need more time to handle (e.g., cut through) a larger amount of tissue. In addition, the predicted surgical case time model 121 may determine a higher case difficulty level based on a larger bone-to-tissue ratio, PPT, and/or PTT determined by the one or more algorithms 90, as a joint (e.g., knee) may be harder to balance due to more tissue.

As an example in the context of a knee surgery, the joint-space width algorithm 50 may determine that a medial space width is narrowing over time and/or is smaller than a predetermined threshold, the osteophyte volume algorithm 60 may determine that an osteophyte volume in the femur is increasing over time, the B-score algorithm 70 may determine that a B-score of the femur is larger (e.g., 3 or greater) than an average B-score for a similarly situated patient, and the alignment/deformity algorithm 80 may determine that the patient has a varus-valgus deformity, and the predicted surgical case time model 121 may predict a longer procedure time 2010 for a total knee arthroplasty.

The one or more algorithms 90 may also determine (or be used by the predicted surgical case time model 121) to determine other aspects of the procedure plan 2020, such as steps, instructions, tools, etc. for preparing for and/or performing a procedure (e.g., surgery). The procedure plan 2020 may include a planned number, position, length, slope, angle, orientation, etc. of one or more tissue incisions or bone cuts, a planned type of the implant, a planned design (e.g., shape and material) of the implant, a planned or target position or alignment of the implant, a planned or target fit or tightness of the implant (e.g., based on gaps and/or ligament balance), a desired outcome (e.g., alignment of joints or bones, bone slopes such as tibial slopes, activity levels, or desired values for postoperative outputs 2000), a list of steps for the surgeon to perform, a list of tools that may be used, etc. The predicted surgical case time model 121 may determine, based on a longer predicted procedure duration 2010, that a type or extent of the procedure in the procedure plan 2020 should include a more corrective surgery, such as from a partial joint (e.g., knee, hip, or shoulder) replacement to a total joint replacement, that certain fixation or other techniques should be used, whether cementing techniques or cementless techniques or implants should be used, etc.

The procedure plan 2020 may, for example, include instructions on how to prepare a proximal end of a tibia to receive a tibial implant, how to prepare a distal end of a femur to receive a femoral implant, how to prepare a glenoid or humerus to receive a glenoid sphere and/or humeral prosthetic component, how to prepare a socket area or acetabulum to receive a ball joint, etc. The bone surface may be cut, drilled, or shaved relative to a reference (e.g., a transepicondylar axis). The procedure plan 2020 may include positions, lengths, and other dimensions for the surfaces and/or values for the slopes for bone preparation. As will be described later, the procedure plan 2020 may be updated and/or modified based on intraoperative data 3000.

The procedure plan 2020 may also include predictive or target outcomes and/or parameters, such as target postoperative range of motion and alignment parameters, and target scores (e.g., stability, fall risk, joint stiffness or laxity, or OA progression). These target parameters may ultimately be compared postoperatively to corresponding measured postoperative data or results to determine whether an optimized outcome for a patient was achieved. The predicted surgical case time model 121 may be configured to update the procedure plan 2020 based on manual input and/or feedback input by practitioners, newly acquired preoperative data 1000, or patient feedback.

The predicted surgical case time model 121 may determine, based on a joint-space width determined by the joint-space width algorithm 50 and/or alignment/deformity parameters determined by the alignment/deformity algorithm 70, that the procedure plan 2020 should include a certain implant design or dimensions. For example, based on a determined joint-space width or joint-space narrowing by the joint-space width algorithm 50, the predicted surgical case time model 121 may determine that an implant width should be decreased and/or determine a type of implant (e.g., a constrained type) based on a narrower determined joint-space width or joint-space narrowing. Based on a joint width and/or an increased joint-space width determined by the joint-space width algorithm 50 and/or a looser or less stable joint determined by alignment/deformity algorithm 70, the predicted surgical case time model 121 may determine that an implant width should be increased (e.g., with augments or shims) and/or determine a type of implant should be a stabilizing or constrained type of implant, that a type or extent of procedure in the procedure plan 2020 should include a more corrective surgery, such as from a partial joint (e.g., knee, hip, or shoulder) replacement to a total joint replacement, etc.

The predicted surgical case time model 121 may determine (or be used to determine) that the predicted outcomes 2080 may include a certain perceived pain level, a predicted stress level, anxiety level, and/or mental health status of the patient, a certain recovery time, certain risks of infection, certain risks of complications during a procedure (e.g., breathing difficulties and/or blood flow or heart rate complications), certain risks or likelihood of revision surgery, and a rating of difficulty for a case. With respect to cartilage loss, the predicted surgical case time model 121 may determine a Z score or other statistical measure to determine a risk of cartilage loss. The determined predicted cartilage loss may be based on the joint space width.

For example, the predicted surgical case time model 121 may predict an increased perceived pain level, a predicted stress level, anxiety level, and/or mental health status of the patient, an increased recovery time, an increased risk of complications during the procedure, an increased risk of infection, an increased likelihood of revision surgery, and/or an increased difficulty rating based on a comparison of the determined joint space width by the joint space width algorithm 50 with a planned implant size in the determined procedure plan 2020, based on a narrower joint space width determined by the joint space width algorithm 50, based on joint space narrowing over time determined by the joint space width algorithm 50, based on a larger osteophyte volume or osteophyte number determined by the osteophyte volume algorithm 60, based on an increasing or progressing osteophyte volume determined by the osteophyte volume algorithm 60, based on a higher or increasing B-score (or alternatively, a B-score outside of a predetermined range) determined by the B-score algorithm 70, based on a severe deformity detected by the alignment/deformity algorithm 80, based on an OA progression determined using the one or more algorithms 90, based on impingement data calculated using parameters determined from the joint space width algorithm 50, the osteophyte volume algorithm 60, and/or the alignment/deformity algorithm 80, a larger bone-to-tissue ratio, PPT, and/or PTT, etc.

Similarly, the predicted surgical case time model 121 may predict a decrease perceived pain level, a decreased stress level or anxiety level of the patient, and increased mental health status of the patient, a decreased recovery time, a decreased risk of complication during the procedure, a decreased risk of infection, a decreased likelihood of revision surgery, and/or a decreased difficulty rating based on a comparison of the determined joint space width by the joint space width algorithm 50 with a planned implant size in the determined procedure plan 2020, based on a joint space width within a predetermined range determined by the joint space width algorithm 50, based on a slower joint space narrowing or widening over time and/or a joint space remaining constant over time determined by the joint space width algorithm 50, based on a lower osteophyte volume or osteophyte number determined by the osteophyte volume algorithm 60, based on a slower progressing and/or constant osteophyte volume determined by the osteophyte volume algorithm 60, based on a lower and/or constant B-score determined by the B-score algorithm 70, based on a healthier alignment and/or a less severe deformity detected by the alignment/deformity algorithm 80, based on a lower OA progression determined using the one or more algorithms 90, based on impingement data calculated using parameters determined from the joint space width algorithm 50, the osteophyte volume algorithm 60, and/or the alignment/deformity algorithm 80, a smaller bone-to-tissue ratio, PPT, and/or PTT, etc.

The predicted surgical case time model 121 may also determine, assign, and/or designate assigned staff 2020 to assist in performance of the procedure. For example, the predicted surgical case time model 121 may determine that the assigned staff 2020 should include surgeons, nurses, or other individuals having more experience with a type of surgery (e.g., knee surgery or total knee arthroplasty) planned in the procedure plan 2020 and/or having more experience with patients having similar characteristics as the instant patient (e.g., narrower joint space width, patient history, a certain type of deformity etc.) The predicted surgical case time model 121 may determine that the assigned staff 2020 should include surgeons, nurses, or other individuals having experience with procedures that take as long as the predicted procedure time 2010. The predicted surgical case time model 121 may store or determine experience scores or levels for each staff member, and may determine an average of a composite procedure or staff team and/or use a rolling average to determine the assigned staff 2020.

The predicted surgical case time model 121 may determine that the assigned staff 2020 should have, individually and/or collectively, more experience based on: a certain type or more complex implant plan, a narrower (or narrowing over time) joint space width determined by the joint space width algorithm 50, a larger osteophyte volume or osteophyte number (or increasing osteophyte volume or number over time, or an osteophyte volume outside of a predetermined range) determined by the osteophyte volume algorithm 60, a higher (or increasing) B-score determined by the B-score algorithm 70, a severe or complicated deformity detected by the alignment/deformity algorithm 80, an OA progression determined using the one or more algorithms 90, impingement data calculated using parameters determined from the joint space width algorithm 50, the osteophyte volume algorithm 60, and/or the alignment/deformity algorithm 80, etc.

The predicted surgical case time model 121 may also determine an operating room layout 2030 and an operating room schedule 2040 based on joint-space width parameters determined by the joint-space width algorithm 50, osteophyte volume parameters determined by the osteophyte volume algorithm 60, B-score determined by the B-score algorithm 70, a bone-to-tissue ratio, PPT, and/or PTT, and/or based on the predicted procedure time 2010 or other determinations or outputs 2000 (e.g., assigned staff 2050). The OR layout 2030 may include a room size, a setup, an orientation, starting location, positions and/or a movement or movement path of certain objects or personnel such as robotic device 142, a practitioner, surgeon or other staff member, operating room table, cameras, displays 210, other equipment, sensors, or patient. The predicted surgical case time model 121 may determine a series of alerts, warnings, and/or reminders sent to practitioners, hospital staff, and/or patients in preparation for the operation and/or during the operation. The predicted surgical case time model 121 may determine or output a new alert to practitioners, hospital staff, and/or patients based on a change in any of the previously determined outputs 2000, which may be based on newly acquired preoperative data 1000 and/or intraoperative data 3000 described later. In some examples, an alert may be a message or indication displayed on a graphical user interface preoperatively or intraoperatively.

For example, the predicted surgical case time model 121 may schedule a longer surgery time based on a longer predicted procedure time 2010 (and/or parameters associated with a longer procedure time 2010, such as a narrower joint space width, a larger osteophyte volume, a larger B-score, a deformity, a larger bone-to-tissue ratio, PPT, and/or PTT, etc.), and may determine certain relative positions of staff and/or equipment in the operating room layout 2030 based on determined assigned staff 2050 and/or tools to use as part of the determined procedure plan 2020. The predicted surgical case time model 121 may also use surgeon data 1040, planned procedure data 1030, and/or other data (e.g., a hospital's operating room schedule and/or floor plan) to determine an operating room layout 2030 and an operating room schedule 2040. The predicted surgical case time model 121 may optimize the OR layout 203 and/or the operating room schedule 2040 to reduce and/or optimize the predicted procedure time 2010. For example, the predicted surgical case time model 121 may place certain equipment to clear a movement path for staff and/or for the surgical robot 242 to reduce actual time spent during the procedure. The predicted surgical case time model 121 may also determine case management and/or workflow priorities for hospital staff, such as a priority order of case or data processing, based on the other outputs 2000.

The predicted surgical case time model 121 may also determine or be used to determine surgeon ergonomics 2070 guidance. For example, the predicted surgical case time model 121 may recommend certain postures or positions for assigned staff 2050 based on a longer predicted procedure time 2010 (and/or parameters associated with a longer procedure time 2010, such as a narrower joint space width, a larger osteophyte volume, a larger B-score, a more severe deformity, a larger bone-to-tissue ratio, PPT, and/or PTT, etc.), past experience of the assigned staff 2050, and/or tools to use as part of the determined procedure plan 2020. The predicted surgical case time model 121 may optimize surgeon ergonomics 2070 to reduce and/or optimize the predicted procedure time 2010.

The outputs 2000 may be output electronically (e.g., on display 260 and/or a mobile device 270) or printed physically (e.g., on paper, canvas, or film 280 or other materials via a printer). The display 260 may include a plurality of screens and/or graphical user interfaces 290 to output the outputs 2000. In some embodiments, the predicted surgical case time model 121, or one or more other system, may also determine intra-operative steps and/or workflows. For example, the predicted surgical case time model 121 may recommend a particular subset of steps that may optimize the procedure workflow and/or minimize the time necessary to complete the operation. In some embodiments, any of the systems described herein may include a deep learning model (a machine-learning model) with osteophyte data/information to predict intra-operative (intra-op) procedure steps and workflows. A deep learning model may be trained on CT data, including but not limited to CT images or features derived from CT images. The deep learning model may output updates to pre-operative, intra-op, and/or post-operative procedure steps and/or updates to procedure workflows based on real-time data, patient data collected prior to the procedure, and/or prior data from one or more patients with one or more similar conditions to the current patient.

The aforementioned machine-learning model may also incorporate patient information, such as body mass index (BMI), age, gender, or any other type of patient information discussed herein. Such patient information may be encoded in one or more format suitable for processing by the deep learning model. Furthermore, the deep learning model may also incorporate osteophyte data. For example, a deep learning model/machine-learning model may be incorporated into the osteophyte volume algorithm 60, and any of the data discussed herein in relation to the osteophyte volume algorithm 60 may be incorporated into a deep learning model as part of osteophyte volume algorithm 60. For example, as discussed hereinabove, such data may include the location and volume of one or more osteophytes, which may be obtained via CT images or other imaging techniques, as well as additional osteophyte-related parameters. For example, density of osteophytes within a target region may be used by the deep learning model to update a procedure workflow. These osteophyte-related parameters may be determined as described throughout this disclosure, or in some embodiments, by an osteophyte detection module, which may be an integrated component of any of the systems described herein or a separate entity. This osteophyte detection module may use various algorithms or techniques, including but not limited to machine learning, image processing algorithms, or the like, to determine the location and volume of osteophytes from CT images or other types of medical images.

The resulting trained deep learning model, with the integration of CT data, patient information, and osteophyte data, may provide enhanced predictions. These enhanced predictions may include, but are not limited to, the sequence of intra-op steps, the estimated duration of each step, the potential complications that may arise during surgery, or the like. Furthermore, such trained models may also predict the balancing workflow, which may assist in intra-op decision making. The prediction of the balancing workflow may be based on various factors, such as the presence and extent of osteophytes, the patient's BMI, age, gender, or the like.

These factors may be processed by one or more modules or systems described herein, and/or by a balancing workflow prediction module, which may be an integrated component of the system or a separate entity. The balancing workflow prediction module may utilize the output of the trained deep learning model to predict the most appropriate balancing workflow for a given patient, thereby assisting in intra-op decision making. While the above description focuses on deep learning models, other types of machine learning models or statistical models may also be used.

Joint balancing may be performed during a total knee arthroplasty (TKA) procedure, or any other orthopedic procedure, and may be performed prior to resection of the patient (e.g. prior to a tibial cut, etc.), and/or mid-resection or after a first tibial cut or other resection. In some examples, one or more of the systems described herein may determine the presence and/or extend of osteophytes present within a target area, and adjust when joint balancing will occur during a medical procedure. For example, intra-operative updates to a surgical plan may occur mid-resection and may change a surgical plan to include additional joint balancing steps based on intraoperative data, such as intraoperative osteophyte data.

In some examples, ligament integrity may be assessed prior to and/or during a medical procedure. For example, one or more algorithms may use an assessment of ligament integrity, such as how many osteophytes are present on a ligament or the degree of calcification of a ligament, to determine what type of implant to use in a medical procedures. In some examples, one or more algorithms may determine whether to use a posterior stabilizing (PS) implant or a cruciate retaining (CR) implant based on an assessment of ligament integrity or based on any other assessment and/or data discussed herein.

In some examples, as discussed hereinabove, deformity may be determined using CT image data. For example, based on the presence of osteophytes, the amount of coronal deformity correction that can occur due to removal of osteophytes may be predicted by one or more algorithms in one or more systems discussed herein. In some examples, a quantity of osteophyte removal may be determined based on a deformity correction algorithm, which may utilize any of the patient data discussed herein. In some examples, CT image data may indicate one or more flexion contractures, and a surgical plan may be updated to account for the detected flexion contractures. In some examples, a surgical plan may be adjusted to reduce flexion contractures, such as by additional removal of osteophytes, adjustments to resection lengths and angles, and implant selection and size adjustments.

Further examples of the predicted surgical case time model are disclosed in U.S. App. No. 18/338,102, the disclosure of which is incorporated herein by reference in its entirety.

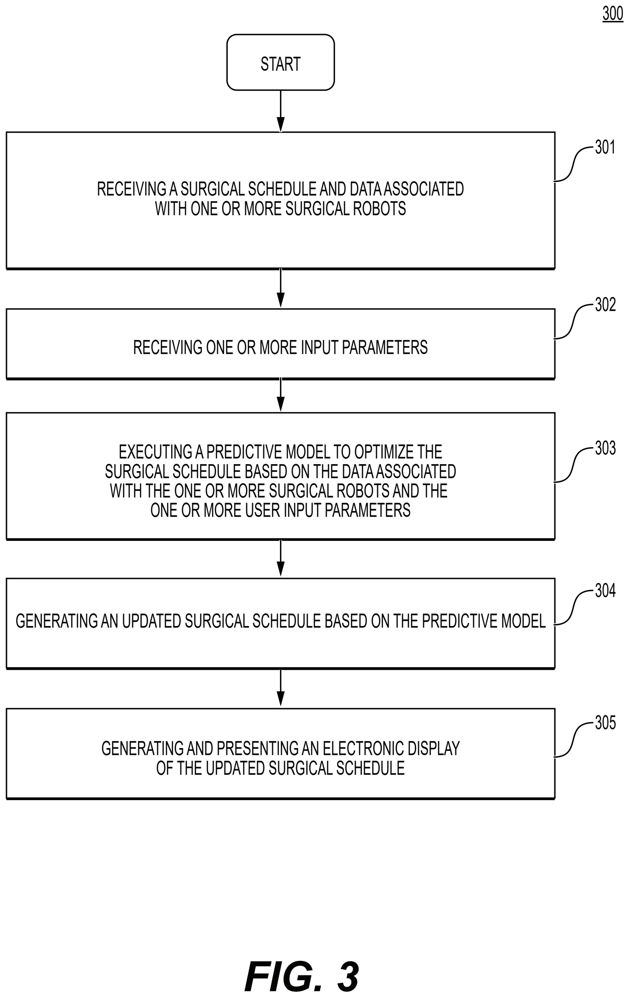

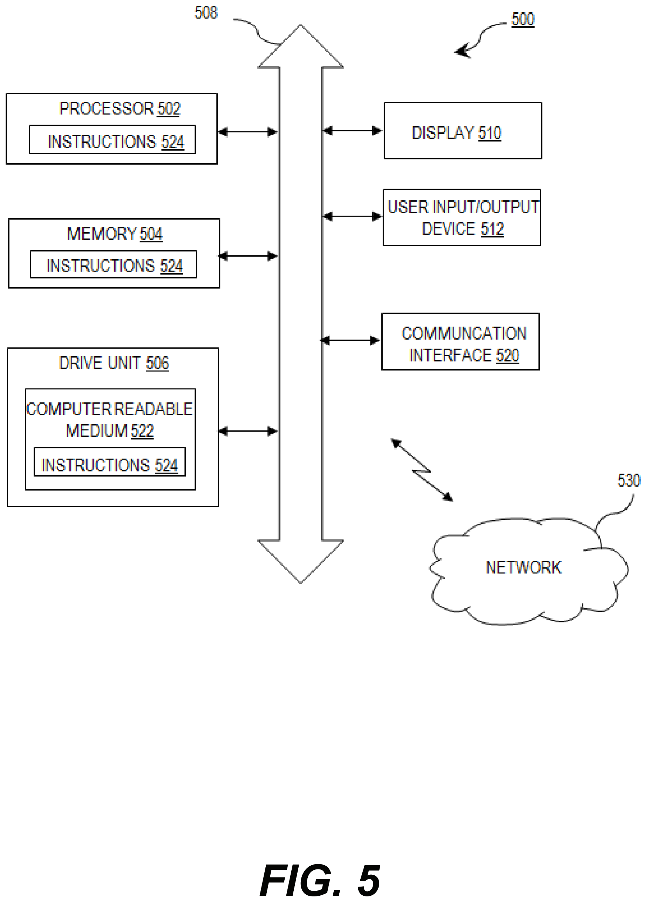

Fig. 3 is an exemplary flowchart of a computer-implemented or computer-based process 300 for optimizing scheduling and inventory associated with medical procedures. In one instance, the predictive scheduling model 140 and/or any of the systems described in Fig. 1 may perform one or more portions of the process 300 and are implemented using, for instance, a chip set including a processor (e.g., processor 502) and a memory (e.g., memory 504) as shown in Fig. 5. As such, the predictive scheduling model 140 and/or any of the systems described in Fig. 1 may be configured to facilitate accomplishing various parts of the process 300, as well as accomplishing embodiments of other processes described herein in conjunction with other components of the system 100. Although the process 300 is illustrated and described as a sequence of actions, operations, and/or functionality, it is contemplated that various embodiments of the process 300 may be performed in any order or combination and need not include all of the illustrated actions, operations, and/or functionality.

In block 301, the process 300 includes receiving a surgical schedule and data associated with one or more surgical robots. The data associated with one or more surgical robots may include, for example, the location of the surgical robots within a facility or within a number of facilities, the number of available surgical robots, the types of surgical robots available (e.g., the procedures that may be performed by the available surgical robots), other equipment necessary for the use of a surgical robot (for example, whether the surgical robot requires a guidance cart or not), staff necessary for the operation of the surgical robot (in addition to the surgeon, there may be other facility staff required for the operation of a surgical robot, or staff from the medical device provider that provides a facility with the surgical robot). Other data may be received associated with the one or more surgical robots as well. In some instances, the surgical schedule includes one or more medical procedures at one or more facilities.

In block 302, the process 300 includes receiving one or more input parameters. In some instances, the one or more input parameters includes data associated with a surgical tool. The data associated with the surgical tool may include, for example, the location of the surgical tool within a facility or within a number of facilities, the number of available surgical tools, the types of surgical tools available, the procedures that may be performed using the available surgical tools, other equipment necessary for the use of a surgical tool (for example, whether the surgical robot requires a tray or not, or whether an instrument requires a complete tray), staff necessary for the operation of the surgical tool (in addition to the surgeon, there may be other facility staff required for the operation of a surgical tool, or staff from the medical device provider that provides a facility with the surgical tool), status of tools with regard to sterilization (e.g., ready to use, in use, or being sterilized).

In some instances, the one or more input parameters includes trend data associated with one or more medical procedures associated with the surgical schedule, wherein the trend data includes one or more of patient growth rate, medical procedure growth rate, and surgical robot use growth rate. The trend data may also be used to determine when a facility will reach its surgical capacity. The trend data may be based on growth rates that measure, e.g., the growth of facility volume, facility equipment, number of surgeries to be demanded, number of surgeries available, or a combination of any or all of the above. Facility volume may include a total number of operating rooms, beds, etc. at a given facility, e.g., a hospital or clinic, and may also include a number of cases per time period performed at the facility. Facility equipment may include surgical robots, guidance carts, tools and their trays, and other equipment available at a facility to aid in the performance of surgical procedures. The number of surgeries demanded may be a value dependent on a variety of external factors and may be predicted into the future based on, for example, the demographics of a surrounding area of a facility, past trends extrapolated into the future, and predictions of changes based on other external factors. The number of surgeries available may be a value reflecting trends in growth in facility volume, number of surgeons available, and improvements in time per procedure based on extrapolated data or predictions based on changes in technology, etc. Trend data may reflect, for example, an increase in procedure volume for a specific type of procedure, such as total knee arthroplasty, an increase in procedures involving the use of surgical robots generally, etc.

In block 303, the process 300 includes executing a predictive model to optimize the surgical schedule based on the data associated with the one or more surgical robots and the one or more user input parameters. In some instances, executing the predictive model is performed to predict scheduling and inventory requirements based on the surgical schedule and the data associated with the surgical tool. In some instances, executing the predictive model is performed to predict scheduling and inventory requirements based on the surgical schedule and the trend data. In other examples, executing the predictive model is performed to predict the inventory requirements based on (i) the surgical schedule, (ii) the data associated with the one or more surgical robots, (iii) the data associated with one or more surgical tool, (iv) some combination of factors (i)-(iii), or based on other factors.

In block 304, the process 300 includes generating an updated surgical schedule based on the predictive model. In some instances, generating inventory requirements based on the predictive model. In some instances, the process 300 includes generating and presenting an electronic display of the updated surgical schedule. In some instances, the process 300 includes generating a digital file that may be read into existing hospital scheduling systems. In some instances, the process 300 includes generating and presenting an electronic display of the inventory requirements.

In block 305, the process 300 includes generating and presenting an electronic display of the updated surgical schedule. In some instances, block 305 also includes generating and presenting an electronic display of the updated surgical schedule and inventory requirements.

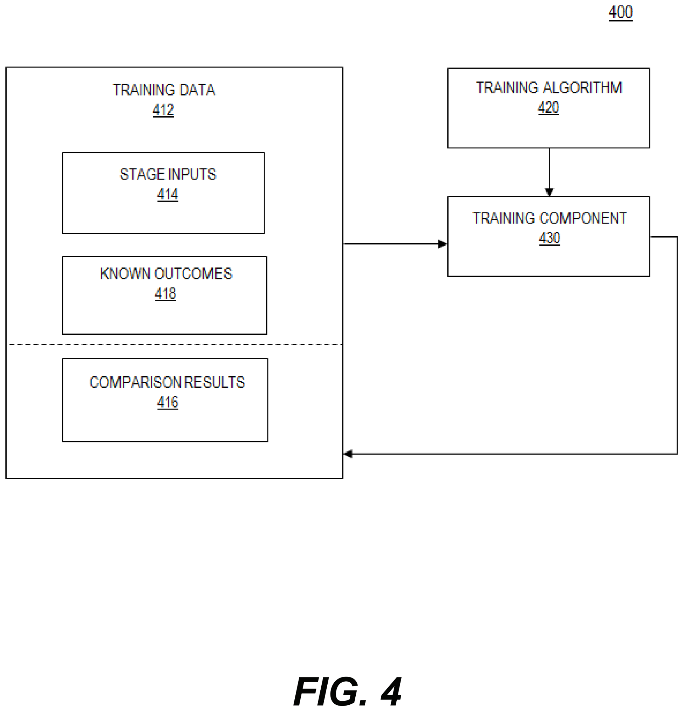

One or more implementations disclosed herein include and/or may be implemented using a machine learning model. For example, the predictive scheduling model 140 may be implemented using a machine learning model and/or may be used to train the machine learning model or other optimization method (e.g., a genetic algorithm). A given machine learning model may be trained using the data flow 400 of Figure 4. Training data 412 may include one or more of stage inputs 414 and known outcomes 418 related to the machine learning model to be trained. The stage inputs 414 may be from any applicable source including text, visual representations, data, values, comparisons, stage outputs, e.g., one or more outputs from one or more actions or operations from Figures 1-3. The known outcomes 418 may be included for the machine learning models generated based upon supervised or semisupervised training. Known outcomes 418 may include known or desired outputs for future inputs similar to or in the same category as stage inputs 414 that do not have corresponding known outputs.

The training data 412 and a training algorithm 420, e.g., one or more of the modules implemented using the machine learning model and/or may be used to train the machine learning or optimization model, may be provided to a training component 430 that may apply the training data 412 to the training algorithm 420 to generate the machine learning model. According to an implementation, the training component 430 may be provided comparison results 416 that compare a previous output of the corresponding machine learning model to apply the previous result to re-train the machine learning model. The comparison results 416 may be used by training component 430 to update the corresponding machine learning model. The training algorithm 420 may utilize machine learning networks and/or models including, but not limited to a deep learning network such as Deep Neural Networks (DNN), Convolutional Neural Networks (CNN), Fully Convolutional Networks (FCN) and Recurrent Neural Networks (RCN), probabilistic models such as Bayesian Networks and Graphical Models, classifiers such as K-Nearest Neighbors, and/or discriminative models such as Decision Forests and maximum margin methods, models specifically discussed in the present disclosure, or the like.

The machine learning model used herein may be trained and/or used by adjusting one or more weights and/or one or more layers of the machine learning model. For example, during training, a given weight may be adjusted (*e.g.*, increased, decreased, removed) based upon training data or input data. Similarly, a layer may be updated, added, or removed based upon training data/and or input data. The resulting outputs may be adjusted based upon the adjusted weights and/or layers.

In general, any process or operation discussed in this disclosure is understood to be computer-implementable, such as the processes illustrated in Figs. 1-3 may be performed by one or more processors of a computer system as described herein. A process or process action or operation performed by one or more processors may also be referred to as an operation. The one or more processors may be configured to perform such processes by having access to instructions (e.g., software or computer-readable code) that, when executed by one or more processors, cause the one or more processors to perform the processes. The instructions may be stored in a memory of the computer system. A processor may be a central processing unit (CPU), a graphics processing unit (GPU), or any suitable type of processing unit.

A computer system, such as a system or device implementing a process or operation in the examples above, may include one or more computing devices. One or more processors of a computer system may be included in a single computing device or distributed among a plurality of computing devices. One or more processors of a computer system may be connected to a data storage device. A memory of the computer system may include the respective memory of each computing device of the plurality of computing devices.

In general, any process or operation discussed in this disclosure is understood to be computer-implementable, such as the processes illustrated in Figs. 1-4 and may be performed by one or more processors of a computer system as described herein. A process or process action or operation performed by one or more processors may also be referred to as an operation. The one or more processors may be configured to perform such processes by having access to instructions (*e.g.*, software or computer-readable code) that, when executed by one or more processors, cause the one or more processors to perform the processes. The instructions may be stored in a memory of the computer system. A processor may be a central processing unit (CPU), a graphics processing unit (GPU), or any suitable type of processing unit.

A computer system, such as a system or device implementing a process or operation in the examples above, may include one or more computing devices. One or more processors of a computer system may be included in a single computing device or distributed among a plurality of computing devices. One or more processors of a computer system may be connected to a data storage device. A memory of the computer system may include the respective memory of each computing device of the plurality of computing devices.

Fig. 5 illustrates an implementation of a computer system that may execute techniques presented herein. The computer system 500 can include a set of instructions that can be executed to cause the computer system 500 to perform any one or more of the methods or computer based functions disclosed herein. The computer system 500 may operate as a standalone device or may be connected, e.g., using a network, to other computer systems or peripheral devices.

Unless specifically stated otherwise, as apparent from the following discussions, it is appreciated that throughout the specification, discussions utilizing terms such as "processing," "computing," "calculating," "determining", "analyzing" or the like, refer to the action and/or processes of a computer or computing system, or similar electronic computing device, that manipulate and/or transform data represented as physical, such as electronic, quantities into other data similarly represented as physical quantities.

In a similar manner, the term "processor" may refer to any device or portion of a device that processes electronic data, *e.g.,* from registers and/or memory to transform that electronic data into other electronic data that, *e.g.,* may be stored in registers and/or memory. A "computer," a "computing machine," a "computing platform," a "computing device," or a "server" may include one or more processors.

In a networked deployment, the computer system 500 may operate in the capacity of a server or as a client user computer in a server-client user network environment, or as a peer computer system in a peer-to-peer (or distributed) network environment. The computer system 500 can also be implemented as or incorporated into various devices, such as a personal computer (PC), a tablet PC, a personal digital assistant (PDA), a mobile device, a palmtop computer, a laptop computer, a desktop computer, a web appliance, or any other machine capable of executing a set of instructions (sequential or otherwise) that specify actions to be taken by that machine. In a particular implementation, the computer system 500 may be implemented using electronic devices that provide voice, video, or data communication. Further, while the computer system 500 is illustrated as a single system, the term "system" shall also be taken to include any collection of systems or sub-systems that individually or jointly execute a set, or multiple sets, of instructions to perform one or more computer functions.

As illustrated in Fig. 5, the computer system 500 may include a processor 502, e.g., a central processing unit (CPU), a graphics processing unit (GPU), or both. The processor 502 may be a component in a variety of systems. For example, the processor 502 may be part of a standard personal computer or a workstation. The processor 502 may be one or more processors, digital signal processors, application specific integrated circuits, field programmable gate arrays, servers, networks, digital circuits, analog circuits, combinations thereof, or other now known or later developed devices for analyzing and processing data. The processor 502 may implement a software program, such as code generated manually (*i.e.,* programmed).

The computer system 500 may include a memory 504 that can communicate via bus 508. The memory 504 may be a main memory, a static memory, or a dynamic memory. The memory 504 may include, but is not limited to computer readable storage media such as various types of volatile and non-volatile storage media, including but not limited to random access memory, read-only memory, programmable read-only memory, electrically programmable read-only memory, electrically erasable read-only memory, flash memory, magnetic tape or disk, optical media and the like. In one implementation, the memory 504 may include a cache or random-access memory for the processor 502. In alternative implementations, the memory 504 is separate from the processor 502, such as a cache memory of a processor, the system memory, or other memory.

The memory 504 may be an external storage device or database for storing data. Examples include a hard drive, compact disc ("CD"), digital video disc ("DVD"), memory card, memory stick, floppy disc, universal serial bus ("USB") memory device, or any other device operative to store data. The memory 504 is operable to store instructions executable by the processor 502. The functions, acts or tasks illustrated in the figures or described herein may be performed by the processor 502 executing the instructions stored in the memory 504. The functions, acts, or tasks are independent of the particular type of instruction set, storage media, processor or processing strategy and may be performed by software, hardware, integrated circuits, firmware, micro-code and the like, operating alone or in combination. Likewise, processing strategies may include multiprocessing, multitasking, parallel processing, and the like.

As shown, the computer system 500 may further include a display 510, such as a liquid crystal display (LCD), an organic light emitting diode (OLED), a flat panel display, a solid-state display, a cathode ray tube (CRT), a projector, a printer or other now known or later developed display device for outputting determined information. The display 510 may act as an interface for the user to see the functioning of the processor 502, or specifically as an interface with the software stored in the memory 504 or in the drive unit 506.

Additionally or alternatively, the computer system 500 may include an input/output device 512 configured to allow a user to interact with any of the components of the computer system 500. The input/output device 512 may be a number pad, a keyboard, or a cursor control device, such as a mouse, or a joystick, touch screen display, remote control, or any other device operative to interact with the computer system 500.

The computer system 500 may also or alternatively include drive unit 506 implemented as a disk or optical drive. The drive unit 506 may include a computer-readable medium 522 in which one or more sets of instructions 524, *e.g.,* software, can be embedded. Further, instructions 524 may embody one or more of the methods or logic as described herein. The instructions 524 may reside completely or partially within the memory 504 and/or within the processor 502 during execution by the computer system 500. The memory 504 and the processor 502 also may include computer-readable media as discussed above.

In some systems, computer-readable medium 522 includes the set of instructions 524 or receives and executes the set of instructions 524 responsive to a propagated signal so that a device connected to network 530 can communicate voice, video, audio, images, or any other data over the network 530. Further, the set of instructions 524 may be transmitted or received over the network 530 via communication port or interface 520, and/or using bus 508. The communication port or interface 520 may be a part of the processor 502 or may be a separate component. The communication port or interface 520 may be created in software or may be a physical connection in hardware.

The communication port or interface 520 may be configured to connect with a network 530, external media, the display 510, or any other components in computer system 500, or combinations thereof. The connection with the network 530 may be a physical connection, such as a wired Ethernet connection or may be established wirelessly as discussed below. Likewise, the additional connections with other components of the computer system 500 may be physical connections or may be established wirelessly. The network 530 may alternatively be directly connected to the bus 508.

While the computer-readable medium 522 is shown to be a single medium, the term "computer-readable medium" may include a single medium or multiple media, such as a centralized or distributed database, and/or associated caches and servers that store one or more sets of instructions. The term "computer-readable medium" may also include any medium that is capable of storing, encoding, or carrying a set of instructions for execution by a processor or that causes a computer system to perform any one or more of the methods or operations disclosed herein. The computer-readable medium 522 may be non-transitory, and may be tangible.

The computer-readable medium 522 can include a solid-state memory such as a memory card or other package that houses one or more non-volatile read-only memories. The computer-readable medium 522 can be a random-access memory or other volatile re-writable memory. Additionally or alternatively, the computer-readable medium 522 can include a magneto-optical or optical medium, such as a disk or tapes or other storage device to capture carrier wave signals such as a signal communicated over a transmission medium. A digital file attachment to an e-mail or other self-contained information archive or set of archives may be considered a distribution medium that is a tangible storage medium. Accordingly, the disclosure is considered to include any one or more of a computer-readable medium or a distribution medium and other equivalents and successor media, in which data or instructions may be stored.

In an alternative implementation, dedicated hardware implementations, such as application specific integrated circuits, programmable logic arrays and other hardware devices, can be constructed to implement one or more of the methods described herein. Applications that may include the apparatus and systems of various implementations can broadly include a variety of electronic and computer systems. One or more implementations described herein may implement functions using two or more specific interconnected hardware modules or devices with related control and data signals that can be communicated between and through the modules, or as portions of an application-specific integrated circuit. Accordingly, the present system encompasses software, firmware, and hardware implementations.

Computer system 500 may be connected to network 530. The network 530 may define one or more networks including wired or wireless networks. The wireless network may be a cellular telephone network, an 802.10, 802.16, 802.20, or WiMAX network. Further, such networks may include a public network, such as the Internet, a private network, such as an intranet, or combinations thereof, and may utilize a variety of networking protocols now available or later developed including, but not limited to TCP/IP based networking protocols. The network 530 may include wide area networks (WAN), such as the Internet, local area networks (LAN), campus area networks, metropolitan area networks, a direct connection such as through a Universal Serial Bus (USB) port, or any other networks that may allow for data communication.

The network 530 may be configured to couple one computing device to another computing device to enable communication of data between the devices. The network 530 may generally be enabled to employ any form of machine-readable media for communicating information from one device to another. The network 530 may include communication methods by which information may travel between computing devices.

The network 530 may be divided into sub-networks. The sub-networks may allow access to all of the other components connected thereto or the sub-networks may restrict access between the components. The network 530 may be regarded as a public or private network connection and may include, for example, a virtual private network or an encryption or other security mechanism employed over the public Internet, or the like.

A computer-implemented method for optimizing scheduling and inventory associated with medical procedures may be provided. The computer-implemented method may be performed by one or more local or remote processors of a computing system in communication with one or more local or remote data sources.

Although the present specification describes components and functions that may be implemented in particular implementations with reference to particular standards and protocols, the disclosure is not limited to such standards and protocols. For example, standards for Internet and other packet switched network transmission (e.g., TCP/IP, UDP/IP, HTML, HTTP) represent examples of the state of the art. Such standards are periodically superseded by faster or more efficient equivalents having essentially the same functions. Accordingly, replacement standards and protocols having the same or similar functions as those disclosed herein are considered equivalents thereof.

It will be understood that the actions, operations, and/or functionality of computer-implemented methods discussed are performed in one embodiment by an appropriate processor (or processors) of a processing (*i.e.,* computer) system executing instructions (computer-readable code) stored in storage. It will also be understood that the disclosure is not limited to any particular implementation or programming technique and that the disclosure may be implemented using any appropriate techniques for implementing the functionality described herein. The disclosure is not limited to any particular programming language or operating system.

Although the text herein sets forth a detailed description of numerous different embodiments, it should be understood that the legal scope of the invention is defined by the words of the claims set forth at the end of this patent. The detailed description is to be construed as exemplary only and does not describe every possible embodiment, as describing every possible embodiment would be impractical, if not impossible. One could implement numerous alternate embodiments, using either current technology or technology developed after the filing date of this patent, which would still fall within the scope of the claims.

It should also be understood that, unless a term is expressly defined in this patent using the sentence "As used herein, the term ' ' is hereby defined to mean..." or a similar sentence, there is no intent to limit the meaning of that term, either expressly or by implication, beyond its plain or ordinary meaning, and such term should not be interpreted to be limited in scope based upon any statement made in any section of this patent (other than the language of the claims). To the extent that any term recited in the claims at the end of this disclosure is referred to in this disclosure in a manner consistent with a single meaning, that is done for sake of clarity only so as to not confuse the reader, and it is not intended that such claim term be limited, by implication or otherwise, to that single meaning.

Finally, unless a claim element is defined by expressly reciting the word "means" and a function without the recital of any structure, it is not intended that the scope of any claim element be interpreted based upon the application of 35 U.S.C. § 112(f).

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in exemplary configurations may be implemented as a combined structure or component. Similarly, structures and functionality presented as a single component may be implemented as separate components. These and other variations, modifications, additions, and improvements fall within the scope of the subject matter herein.

Additionally, certain embodiments are described herein as including logic or a number of routines, subroutines, applications, or instructions. These may constitute either software (code embodied on a non-transitory, tangible machine-readable medium) or hardware. In hardware, the routines, etc., are tangible units capable of performing certain operations and may be configured or arranged in a certain manner. In exemplary embodiments, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various embodiments, a hardware module may be implemented mechanically or electronically. For example, a hardware module may comprise dedicated circuitry or logic that is permanently configured (e.g., as a special-purpose processor, such as a field programmable gate array (FPGA) or an application-specific integrated circuit (ASIC) to perform certain operations). A hardware module may also comprise programmable logic or circuitry (*e.g.*, as encompassed within a general-purpose processor or other programmable processor) that is temporarily configured by software to perform certain operations. It will be appreciated that the decision to implement a hardware module mechanically, in dedicated and permanently configured circuitry, or in temporarily configured circuitry (*e.g.*, configured by software) may be driven by cost and time considerations.

Accordingly, the term "hardware module" should be understood to encompass a tangible entity, be that an entity that is physically constructed, permanently configured (e.g., hardwired), or temporarily configured (*e.g.*, programmed) to operate in a certain manner or to perform certain operations described herein. Considering embodiments in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple of such hardware modules exist contemporaneously, communications may be achieved through signal transmission (*e.g.*, over appropriate circuits and buses) that connect the hardware modules. In embodiments in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (*e.g.*, a collection of information).

The various operations of exemplary methods described herein may be performed, at least partially, by one or more processors that are temporarily configured (*e.g*., by software) or permanently configured to perform the relevant operations. Whether temporarily or permanently configured, such processors may constitute processor-implemented modules that operate to perform one or more operations or functions. The modules referred to herein may, in some exemplary embodiments, comprise processor-implemented modules.

Similarly, the methods or routines described herein may be at least partially processor-implemented. For example, at least some of the operations of a method may be performed by one or more processors or processor-implemented hardware modules. The performance of certain of the operations may be distributed among the one or more processors, not only residing within a single machine, but deployed across a number of machines. In some example embodiments, the processor or processors may be located in a single location (*e.g*., within a home environment, an office environment or as a server farm), while in other embodiments the processors may be distributed across a number of geographic locations.

Unless specifically stated otherwise, discussions herein using words such as processing," "computing," "calculating," "determining," "presenting," "displaying," or the like may refer to actions or processes of a machine (*e.g*., a computer) that manipulates or transforms data represented as physical (*e.g.*, electronic, magnetic, or optical) quantities within one or more memories (*e.g.*, volatile memory, non-volatile memory, or a combination thereof), registers, or other machine components that receive, store, transmit, or display information.

As used herein any reference to "one embodiment" or "an embodiment" means that a particular element, feature, structure, or characteristic described in connection with the embodiment may be included in at least one embodiment. The appearances of the phrase "in one embodiment" in various places in the specification are not necessarily all referring to the same embodiment.

Some embodiments may be described using the expression "coupled" and "connected" along with their derivatives. For example, some embodiments may be described using the term "coupled" to indicate that two or more elements are in direct physical or electrical contact. The term "coupled," however, may also mean that two or more elements are not in direct contact with each other, but yet still cooperate or interact with each other. The embodiments are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description, and the claims that follow, should be read to include one or at least one and the singular also includes the plural unless it is obvious that it is meant otherwise.

Upon reading this disclosure, those of skill in the art will appreciate still additional alternative structural and functional designs for the approaches described herein. Therefore, while particular embodiments and applications have been illustrated and described, it is to be understood that the disclosed embodiments are not limited to the precise construction and components disclosed herein. Various modifications, changes and variations, which will be apparent to those skilled in the art, may be made in the arrangement, operation and details of the method and apparatus disclosed herein without departing from the spirit and scope defined in the appended claims.

The particular features, structures, or characteristics of any specific embodiment may be combined in any suitable manner and in any suitable combination with one or more other embodiments, including the use of selected features without corresponding use of other features. In addition, many modifications may be made to adapt a particular application, situation or material to the essential scope and spirit of the present invention. It is to be understood that other variations and modifications of the embodiments of the present invention described and illustrated herein are possible in light of the teachings herein and are to be considered part of the spirit and scope of the present invention.

While the preferred embodiments of the invention have been described, it should be understood that the invention is not so limited and modifications may be made without departing from the invention. The scope of the invention is defined by the appended claims, and all devices that come within the meaning of the claims, either literally or by equivalence, are intended to be embraced therein.

It is therefore intended that the foregoing detailed description be regarded as illustrative rather than limiting, and that it be understood that it is the following claims, including all equivalents, that are intended to define the spirit and scope of this invention.

## Claims

1. A computer-implemented method comprising:
receiving, by one or more processors, a surgical schedule and data associated with one or more surgical robots;
receiving, by one or more processors, one or more input parameters;
executing a predictive model, stored in a non-transitory computer-readable storage medium, to optimize a surgical schedule based on the data associated with the one or more surgical robots and the one or more input parameters;
generating, by the one or more processors, an updated surgical schedule based on the predictive model; and
generating and presenting, by the one or more processors, an electronic display of the updated surgical schedule.

2. The computer-implemented method of claim 1, wherein the predictive model is configured to maximize daily operating time per surgical robot.

3. The computer-implemented method of claim 1, wherein the input parameters include one or more difficulty of procedure parameters for one or more procedures, the one or more difficulty of procedure parameters being determined by CT imaging data.

4. The computer-implemented method of claim 1, wherein the input parameters include one or more parameters indicating a stage of a surgical procedure.

5. The computer-implemented method of claim 1, further comprising:
receiving prior procedure data, the prior procedure data including data from a plurality of prior patients sharing at least one characteristic with a current patient;
and determining a predicted duration for a medical procedure based on the received prior procedure data.

6. The computer-implemented method of claim 5, further comprising:
receiving at least one of (i) patient specific data regarding the current patient, (ii) clinical data relating to the current patient, and (iii) surgeon specific data relating to one or more surgeons, wherein determining the predicted duration for the medical procedure is based on the received patient specific data, clinical data, and/or surgeon specific data.

7. The computer-implemented method of claim 1, further comprising:
determining a predicted duration for a medical procedure of a current patient; and
determining, based on the predicted duration for the medical procedure and/or at least one parameter of the current patient's anatomy, an output, the output including at least one of:
an operating room layout,
an operating room schedule, or
at least one staff member to assist in performance of the medical procedure.

8. The computer-implemented method of claim 7, further comprising:
determining, based on the predicted duration for the medical procedure and/or the at least one parameter of the current patient's anatomy, an output, the output including at least one of:
a risk of infection,
a loss of cartilage,
a predicted pain perceived by the patient after the procedure,
a predicted stress level perceived by the patient after the procedure,
a predicted anxiety level perceived by the patient after the procedure, or
a predicted mental health status of the patient after the procedure.

9. The computer-implemented method of claim 1, further comprising:
receiving, by the one or more processors, the surgical schedule including one or more medical procedures at one or more facilities;
receiving, by the one or more processors, data associated with a surgical tool;
executing the predictive model, stored in the non-transitory computer-readable storage medium, to predict scheduling and inventory requirements based on the surgical schedule and the data associated with the surgical tool;
generating, by the one or more processors, an updated surgical schedule and updated inventory requirements based on the predictive model; and
generating and presenting an electronic display of the updated surgical schedule and the updated inventory requirements.

10. A system for optimizing scheduling and inventory associated with medical procedures, comprising:
a computer-readable storage medium storing instructions for generating and presenting an electronic display of a surgical schedule; and
one or more processors configured to execute the instructions to perform a method including:
receiving a surgical schedule and data associated with one or more surgical robots;
receiving one or more input parameters;
executing a predictive model, stored in the computer-readable storage medium, to optimize the surgical schedule based on the data associated with the one or more surgical robots and the one or more input parameters;
generating an updated surgical schedule based on the predictive model; and
generating and presenting an electronic display of the updated surgical schedule.

11. The system of claim 10, the instructions further comprising:
determining, based on a determined predicted duration for the procedure and/or at least one parameter of the patient's anatomy, an output, the output including at least one of:
an operating room layout,
an operating room schedule, or
at least one staff member to assist in performance of the medical procedure.

12. The system of claim 10 or 11, the instructions further comprising:
determining, based on a determined predicted duration for the procedure and/or at least one parameter of the patient's anatomy, an output, the output including at least one of:
a risk of infection,
a loss of cartilage,
a predicted pain perceived by the current patient after the medical procedure,
a predicted stress level perceived by the current patient after the medical procedure,
a predicted anxiety level perceived by the current patient after the medical procedure, or
a predicted mental health status of the current patient after the medical procedure.

13. The system of claim 10, 11 or 12, the instructions further comprising:
receiving, by the one or more processors, a surgical schedule including one or more medical procedures at one or more facilities;
receiving, by the one or more processors, data associated with a surgical tool;
executing the predictive model, stored in the non-transitory computer-readable storage medium, to predict scheduling and inventory requirements based on the surgical schedule and the data associated with the surgical tool;
generating, by the one or more processors, an updated surgical schedule and inventory requirements based on the predictive model; and
generating and presenting an electronic display of the updated surgical schedule and inventory requirements.

14. A computer-implemented method for generating scheduling and inventory predictions, the method comprising:
receiving, by one or more processors, a surgical schedule including one or more medical procedures;
receiving, by one or more processors, trend data associated with one or more medical procedures associated with the surgical schedule, wherein the trend data includes one or more of patient growth rate, medical procedure growth rate, and surgical robot use growth rate;
executing a predictive model, stored in a non-transitory computer-readable storage medium, to predict scheduling and inventory requirements based on the surgical schedule and the trend data;
generating, by the one or more processors, an updated surgical schedule and updated inventory requirements based on the predictive model; and
generating and presenting an electronic display of the updated surgical schedule and updated inventory requirements.

15. The computer-implemented method of claim 14, wherein the one or more medical procedures include procedures using surgical robots, and the predictive model is configured to maximize daily operating time per surgical robot.
